# EUROPEAN PATENT SPECIFICATION

(11) **EP 1 878 732 B1**
(45) Date of publication and mention of the grant of the patent: **25.02.2009**
(21) Application number: 06732284.2
(22) Date of filing: 24.04.2006
(51) Int. Cl.: C07D 401/12, A61K 31/551, A61P 9/04, A61P 9/10, A61P 9/12, A61P 11/06, A61P 13/02, A61P 15/10, A61P 25/08, A61P 27/06, A61P 43/00

(54) **HIGHLY SELECTIVE Rho-KINASE INHIBITOR**
HOCHSELEKTIVER RHO-KINASE-INHIBITOR
INHIBITEUR HAUTEMENT SELECTIF DE LA Rho-KINASE

(30) Priority: 25.04.2005 JP 2005126423
(43) Date of publication of application: 16.01.2008
(73) Proprietor: D. Western Therapeutics Institute, Inc., Nagoya-shi, Aichi 460-0003 (JP)
(72) Inventor: HIDAKA, Hiroyoshi, 4680063 (JP); TAMURA, Masahiro, 1940003 (JP); NAKAO, Hiroshi, 3000813 (JP); SIGYO, Hiromichi, 1830035 (JP); YAMADA, Hajime 2-17-43-107, Noguchi-cyo,, 1890022 (JP); OZAWA, Takatoshi, 1520034 (JP); YOSHIZAKI, Hideo, 3501315 (JP)
(74) Representative: Jones, Helen M.M.
(86) International application number: PCT/JP2006/308574
(87) International publication number: WO 2006/115247

(56) References cited:
- EP-A1- 0 885 888
- WO-A1-99/20620
- JP-A- 10 087 491
- JP-A- 10 310 576
- JP-A- 2004 155 661
- US-A1- 2005 020 623

## Description

### Technical Field

The present invention relates to a compound having a highly selective Rho-kinase inhibiting activity and being useful as a therapeutic agent for a disease such as hypertension, pulmonary hypertension, cerebral vasospasm, cardiac angina, cardiac failure, arteriosclerosis, glaucoma, dysuria, asthma, or erectile dysfunction, and relates to a drug containing the compound.

### Background Art

Rho is a low-molecular weight GTP-binding protein and is present in cells as active Rho-GTP or inactive Rho-GDP. The inactive Rho-GDP is converted to the active Rho-GTP by Rho-kinase activated by a signal from various cell membrane receptors. It has been reported that the active Rho-GTP functions as a molecular switch of various cellular phenomena through an actomyosin system, such as smooth muscle contraction, cell movement, cell adhesion, cell transformation, and cell propagation. Accordingly, it is conceived that the inhibition of Rho-kinase can suppress responses of various cellular phenomena occurring downstream of signal transduction pathways mediated by Rho and is hence effective in treating diseases in which Rho is involved.

For example, the smooth muscle is contracted by the activation of Rho-kinase and is relaxed by the inhibition of this enzyme. The mechanism of this action is thought that an increase in Ca ion sensitivity mediated by G-protein (guanine nucleotide-binding regulatory protein) is selectively inhibited to decrease Ca ion sensitivity in the cells. It has been reported in papers that Rho-kinase selectively acts on the Ca ion sensitivity-enhancing mechanism, which is one of the smooth muscle contraction mechanisms that do not depend on the intracellular Ca ion concentration (for example, refer to Non-Patent Document 1). Therefore, compounds that inhibit Rho-kinase are considered as promising therapeutic agents having a novel mechanism that works by decreasing Ca ion sensitivity, for example, as therapeutic agents for treating hypertension or the like.

Furthermore, recent researches have reported, in addition to the blood pressure-decreasing effect realized by inhibiting Rho-kinase (for example, refer to Non-Patent Documents 2 and 3), for example, the followings: cases showing effectiveness in pulmonary hypertension (for example, refer to Non-Patent Documents 4 to 6), cases showing effectiveness in cerebral vasospasm (for example, refer to Non-Patent Documents 7 and 8), cases showing effectiveness in cardiac angina (for example, refer to Non-Patent Documents 9 to 11), cases showing effectiveness in glaucoma (for example, refer to Non-Patent Documents 12 to 14), cases showing effectiveness in dysuria (for example, refer to Non-Patent Document 15), cases showing effectiveness in asthma (for example, refer to Non-Patent Documents 16 to 19), and cases showing effectiveness in erectile dysfunction (for example, refer to Non-Patent Documents 20 and 21).

Currently, compounds known as Rho-kinase inhibitors have been disclosed in Patent Documents 1 and 2, for example. However, these compounds inhibit not only Rho-kinase but also other kinases such as protein kinase A (PKA) and protein kinase G (PKG). PKA plays an important role in mediating effects of cAMP in many signal transduction processes. For example, the binding of a hormone to a receptor activates intracellular stimulatory G-protein, leading to activation of an adenylate cyclase. Then, cAMP is produced, and PKA phosphorylates serine/threonine of an enzyme or protein that is involved in intracellular metabolism or transport, resulting in development of physiological functions. PKG also plays an important role in mediating effects of cGMP in many signal transduction processes. For example, in the cardiovascular system, a relaxant substance derived from vascular endothelial cells activates guanylate cyclase to produce cGMP. Then, PKG phosphorylates serine/threonine of intracellular protein to decrease the intracellular Ca concentration, resulting in relaxation of blood vessels and a decrease in blood pressure. Also in nerve cells, a similar pathway is activated to inhibit apoptosis. Thus, PKA and PKG play important roles in cells and living bodies. Accordingly, it is suggested that the inhibition of a PKA or PKG activity may cause severe side effects.

Therefore, from the viewpoint of using a Rho-kinase inhibitor as a therapeutic agent, it has been desired to develop a compound that can selectively inhibit only Rho-kinase involved in a disease, but does not substantially affect activities of other kinases.
Patent Document 1: Japanese Unexamined Patent Application Publication No. 11-349482
Patent Document 2: International Publication No. WO 90/5723
Non-Patent Document 1: Nature 1997, 389, 990.
Non-Patent Document 2: J. Cereb. Blood Flow 2001, 21, 876.
Non-Patent Document 3 : Hypertension 2001, 38, 1307.
Non-Patent Document 4: Atheroscierosis 2003, Supplement 4, 170.
Non-Patent Document 5: Ir. J. Med. Sci. 2003, 172, 20.
Non-Patent Document 6: Circ. Res. 2004, 94, 385.
Non-Patent Document 7: Br. J. Pharmacol. 2000, 130, 219.
Non-Patent Document 8: Stroke 2001, 32, 2913.
Non-Patent Document 9: Jpn. J. Pharmacol. 2001, 87, 34.
Non-Patent Document 10: Br. J. Pharmacol. 2001, 134, 1724.
Non-Patent Document 11: Circulation 2002, 105, 1545.
Non-Patent Document 12: Invest. Ophthalmol. Visual Sci. 2001, 42, 137.
Non-Patent Document 13: Arch. Ophthalmol. 2001, 119.
Non-Patent Document 14: 1171, Invest. Ophthalmol. Visual Sci. 2001, 42, 1029.
Non-Patent Document 15: Br. J. Pharmacol. 2004, 143, 431.
Non-Patent Document 16: Jpn. J. Allergol. 1999, 48, 1079.
Non-Patent Document 17: Eur. J. Pharmacol. 2000, 389, 103.
Non-Patent Document 18: Eur. J. Pharmacol. 2000, 406, 273.
Non-Patent Document 19: Br. J. Pharmacol. 2001, 132, 111.
Non-Patent Document 20: Int. J. Impot. Res. 2001, 13, 67.
Non-Patent Document 21: Br. J. Pharmacol. 2001, 133, 455.

### Disclosure of the Invention

### Problems to Be Solved by the Invention

It is an object of the present invention to provide a compound having a potent and highly selective Rho-kinase inhibiting activity and provide a drug containing the same.

### Means for Solving the Problems

Under the above-mentioned circumstances, the present inventors have conducted intensive studies and, as a result, have found that a compound represented by General Formula (1) below has an excellent Rho-kinase inhibiting activity, but does not substantially inhibit other kinases, and that the compound is useful as a therapeutic agent for a disease such as hypertension, pulmonary hypertension, cerebral vasospasm, cardiac angina, cardiac failure, arteriosclerosis, glaucoma, dysuria, asthma, or erectile dysfunction. Thus, the present invention has been completed.

That is, the present invention provides a homopiperazine derivative represented by General Formula (1), an acid addition salt thereof, or a solvate of the derivative or the salt: 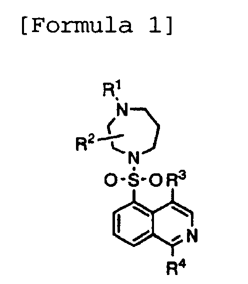 (wherein R¹ represents an amino acid residue; R² represents a hydrogen atom or an alkyl group; R³ represents a hydrogen atom, an alkyl group, an alkenyl group, an alkynyl group, an alkoxy group, a halogen atom, a nitrile group, or a hydroxyl group; and R⁴ represents a hydrogen atom, a halogen atom, or a hydroxyl group).

Furthermore, the present invention provides a drug containing the compound represented by the above General Formula (1), an acid addition salt thereof, or a solvate of the derivative or the salt, as an active ingredient.

Furthermore, the present invention provides a therapeutic agent for a disease caused by activation of Rho-kinase, containing the compound represented by the above General Formula (1), an acid addition salt thereof, or a solvate of the derivative or the salt, as an active ingredient.

Furthermore, the present invention provides a pharmaceutical composition containing the compound represented by the above General Formula (1), an acid addition salt thereof, or a solvate of the derivative or the salt, and a pharmaceutically acceptable carrier.

Furthermore, the present invention provides a use of the compound represented by the above General Formula (1), an acid addition salt thereof, or a solvate of the derivative or the salt, in manufacturing a drug.

Furthermore, the present invention provides a method for treating a disease caused by activation of Rho-kinase by administering the compound represented by the above General Formula (1), an acid addition salt thereof, or a solvate of the derivative or the salt.

### Advantageous Effect of the Invention

The compound (1) according to the present invention has an activity to inhibit Rho-kinase with a high selectivity and is useful as a therapeutic agent for a disease such as hypertension, pulmonary hypertension, cerebral vasospasm, cardiac angina, cardiac failure, arteriosclerosis, glaucoma, dysuria, asthma, or erectile dysfunction.

### Best Mode for Carrying Out the Invention

In General Formula (1), the amino acid residue represented by R¹ may be any residue of a neutral amino acid, an acidic amino acid, and a basic amino acid, and examples thereof include glycyl, sarcosyl, alanyl, 1-aminocyclopropane-1-carbonyl, 1-aminocyclobutane-1-carbonyl, β-alanyl, γ-aminobutyroyl, α-aminobutyryl, valyl, norvalyl, leucyl, isoleucyl, norleucyl, tert-leucyl, phenylalanyl, homophenylalanyl, aspartyl, glutamyl, α,β-diaminopropionyl, α,γ-diaminobutyroyl, ornityl, lysyl, arginyl, homoarginyl, seryl, threonyl, tyrosyl, prolyl, triptophyl, and histidyl. In addition, when the above amino acid residue has an asymmetric carbon, the amino acid residue may be in L- or D-configuration or may be a racemic mixture. Among these, glycyl and sarcosyl are particularly preferred.

In General Formula (1), the alkyl group represented by R² can be a linear or branched alkyl group of C₁-C₆, and examples thereof include methyl, ethyl, n-propyl, isopropyl, n-butyl, isobutyl, sec-butyl, tert-butyl, pentyl, and hexyl.

In General Formula (1), the alkyl group represented by R³ can be a linear or branched alkyl group of C₁-C₆, and examples thereof include the above-mentioned alkyl groups. Among these, a methyl group is particularly preferred.

In General Formula (1), the alkenyl group represented by R³ can be a linear or branched alkenyl group of C₂-C₆, and examples thereof include vinyl, allyl, butenyl, pentenyl, and hexenyl.

In General Formula (1), the alkynyl group represented by R³ can be a linear or branched alkynyl group of C₂-C₆, and examples thereof include ethynyl, propargyl, and butynyl. Among these, an ethynyl group is particularly preferred.

In General Formula (1), the alkoxy group represented by R³ can be a linear or branched alkoxy group of C₁-C₆, and examples thereof include methoxy, ethoxy, n-propoxy, isopropoxy, n-butoxy, isobutoxy, sec-butoxy, tert-butoxy, pentyloxy, and hexyloxy. Among these, a methoxy group is particularly preferred.

In General Formula (1), the halogen atom represented by R³ may be, for example, a fluorine atom, a chlorine atom, a bromine atom, or an iodine atom. Among these, a fluorine atom is particularly preferred.

The substitution position of R³ on the homopiperazine ring may be any of 2-position, 3-position, 5-position, 6-position, and 7-position. The configuration of the substituent may be either (S) or (R). Among these, 2-(S)-methyl is particularly preferred.

In General Formula (1), the halogen atom represented by R⁴ may be, for example, any of the above-mentioned halogen atoms represented by R³.

The acid addition salt of the compound (1) of the present invention is not specifically limited as long as it is pharmaceutically acceptable, and examples thereof include acid addition salts of mineral acids, such as hydrochloride, hydrobromide, hydroiodide, sulfate, and phosphate; and acid addition salts of organic acids, such as benzoate, methanesulfonate, ethanesulfonate, benzenesulfonate, p-toluenesulfonate, oxalate, maleate, fumarate, tartarate, citrate, and acetate.

Furthermore, the compound (1) of the present invention can be in the form of a solvate typified by a hydrate, and such solvates are included in the present invention. In addition, some of the compounds of the present invention exist in the form of cis- or trans-isomers or optical isomers, and such isomers are included in the present invention.

Among the compounds of the present invention, preferable examples that have a high selectivity to Rho-kinase include compounds selected from the group consisting of
2-(S)-1-(4-fluoroisoquinoline-5-sulfonyl)-4-glycyl-2-methylhomopip erazine,
2-(S)-1-(4-fluoroisoquinoline-5-sulfonyl)-2-methyl-4-(N-methylglyc yl)homopiperazine,
2-(S)-4-(L-alanyl)-1-(4-fluoroisoquinoline-5-sulfonyl)-2-methylhom opiperazine,
2-(S)-4-(D-alanyl)-1-(4-fluoroisoquinoline-5-sulfonyl)-2-methylhom opiperazine,
2-(S)-1-(4-fluoroisoquinoline-5-sulfonyl)-2-methyl-4-(L-valyl)homo piperazine,
2-(S)-1-(4-fluoroisoquinoline-5-sulfonyl)-2-methyl-4-(D-valyl)homo piperazine,
2-(S)-1-(4-fluoroisoquinoline-5-sulfonyl)-4-(L-leucyl)-2-methylhom opiperazine,
2-(S)-1-(4-fluoroisoquinoline-5-sulfonyl)-4-(D-leucyl)-2-methylhom opiperazine,
2-(S)-4-(P-alanyl)-l-(4-fluoroisoquinoline-5-sulfonyl)-2-methylhom opiperazine,
2-(S)-4-(1-aminocyclopropane-1-carbonyl)-1-(4-fluoroisoquinoline-5 -sulfonyl)-2-methylhomopiperazine,
2-(S)-4-(L-α-aminobutyryl)-1-(4-fluoroisoquinoline-5-sulfonyl)-2-methylhomopiperazine,
2-(S)-4-(D-a-aminobutyryl)-1-(4-fluoroisoquinoline-5-sulfonyl)-2-methylhomopiperazine,
2-(S)-1-(4-fluoroisoquinoline-5-sulfonyl)-2-methyl-4-(L-norleucyl) homopiperazine,
2-(S)-1-(4-fluoroisoquinoline-5-sulfonyl)-2-methyl-4-(D-norleucyl) homopiperazine,
4-(glycyl)-1-(isoquinoline-5-sulfonyl)homopiperazine,
4-(β-alanyl)-1-(isoquinoline-5-sulfonyl)homopiperazine,
4-(L-alanyl)-1-(isoquinoline-5-sulfonyl)homopiperazine,
4-(D-alanyl)-1-(isoquinoline-5-sulfonyl)homopiperazine,
1-(isoquinoline-5-sulfonyl)-4-(L-valyl)homopiperazine,
1-(isoquinoline-5-sulfonyl)-4-(D-valyl)homopiperazine,
4-(L-α,β-diaminopropionyl)-1-(isoquinoline-5-sulfonyl)homopiperazi ne,
4-(D-α,β-diaminopropionyl)-1-(isoquinoline-5-sulfonyl)homopiperazi ne,
2-(S)-4-(L-α,β-diaminopropionyl)-1-(4-fluoroisoquinoline-5-sulfony 1)-2-methylhomopiperazine,
2-(S)-4-(D-α,β-diaminopropionyl)-1-(4-fluoroisoquinoline-5-sulfony 1)-2-methylhomopiperazine,
4-(glycyl)-1-(4-methylisoquinoline-5-sulfonyl)homopiperazine,
2-(S)-4-(glycyl)-1-(4-methylisoquinoline-5-sulfonyl)-2-methylhomop iperazine,
2-(S)-1-(4-bromoisoquinoline-5-sulfonyl)-4-(glycyl)-2-methylhomopi perazine, and
2-(S)-1-(4-ethenylisoquinoline-5-sulfonyl)-4-(glycyl)-2-methylhomo piperazine; acid addition salts of the above compounds; and solvates of the above compounds or the salts.

The compound (1) of the present invention can be manufactured, for example, by the following method: a compound (4) is prepared by a condensation reaction between a compound (2) and a protected amino acid (3) and then is subjected to a suitable deprotection reaction to give a compound (1) of the present invention. The compound (2) as a starting material for the compound (1) of the present invention can be synthesized by the method described in International Publication No. WO 97/28130, the method described in Japanese Unexamined Patent Application Publication No. 61-227581, or a method similar to these methods. 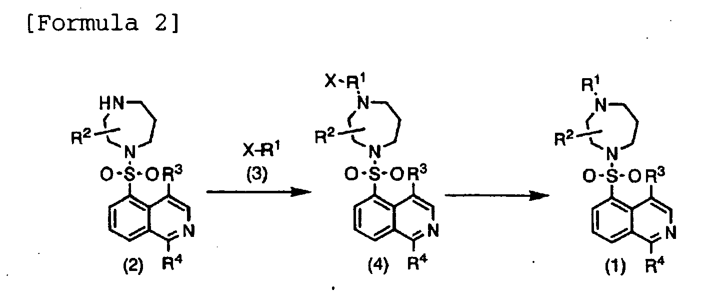 (wherein R¹ to R⁴ represent the same as described above, and X represents an amino-protecting group).

In the above formula, examples of the amino-protecting group, represented by X, include a benzyloxycarbonyl group, a tert-butoxycarbonyl group, a 9-fluorenylmethoxycarbonyl group, a formyl group, an acetyl group, a trifluoroacetyl group, and a trityl group. Furthermore, when the amino acid R¹ has a functional group in a side chain, the functional group is protected by a suitable protecting group.

Each of the above reaction processes will now be described.

The condensation reaction between a compound (2) and a protected amino acid (3) can be performed according to a known method. For example, a reaction using a necessary amount of a condensing agent such as a water-soluble carbodiimide (WSC/HCl), e.g., dicyclohexylcarbodiimide or N-ethyl-N'-(3-dimethylaminopropyl)carbodiimide hydrochloride is carried out under cooling in a solvent. Examples of the solvent include halogenated hydrocarbons such as dichloromethane and chloroform; ethers such as tetrahydrofuran (THF) and diethyl ether; N,N-dimethylformamide (DMF); and acetonitrile. The reaction is preferably carried out in the presence of a necessary amount of an inorganic base such as potassium carbonate, sodium carbonate, or cesium carbonate; or an organic base such as triethylamine, diisopropylethylamine, or triethylenediamine.

The protected amino acid (3) used is preferably 1.0 to 1.5 equivalents to the compound (2).

The reaction is carried out at a temperature of 0°C to the boiling point of the solvent for 1 to 24 hours and preferably at a temperature of 10°C to 30°C for 6 to 12 hours.

The deprotection reaction of the resulting compound (4) can be carried out by a known method, such as acid treatment, alkali treatment, or catalytic reduction, depending on the protecting group. For example, when the protecting group is a tert-butoxycarbonyl group, the compound (1) of the present invention can be obtained by treatment with an ethyl acetate solution of hydrogen chloride.

Furthermore, deprotection is similarly carried out in a case where a functional group in the side chain of the amino acid R¹ is protected with a protecting group.

The compound (1) of the present invention can be further purified, if necessary, by a conventional purification procedure such as recrystallization or column chromatography. Furthermore, a desired salt or solvate mentioned above can be prepared by a conventional method.

The thus obtained compound (1) of the present invention, an acid addition salt, or a solvate of the compound or the salt has a highly selective Rho-kinase inhibiting activity, but is significantly low in inhibitory activities against other kinases such as PKA and PKG, as shown in Test Example 1 described below and, therefore, is useful as a drug for treating a disease such as hypertension, pulmonary hypertension, cerebral vasospasm, cardiac angina, cardiac failure, arteriosclerosis, glaucoma, dysuria, asthma, or erectile dysfunction.

The drug according to the present invention includes a compound (1) of the present invention, a salt thereof, or a solvate of the compound or the salt as an active ingredient. Its dosage form is not specifically limited and can be properly selected depending on the purpose of treatment. For example, any of oral agents, injections, suppositories, ointments, inhalants, ophthalmic solutions, collunaria, and plasters can be employed. Compositions suitable for these administration forms can be prepared by blending a pharmaceutically acceptable carrier with the compound, the salt, or the solvate and by a common drug preparation that is known by those skilled in the art.

A solid preparation for oral administration can be prepared by adding an excipient and, according to need, a binder, a disintegrator, a lubricant, a colorant, a corrigent, or a flavoring agent, to a compound (1) of the present invention, and then forming the mixture into a tablet, a coated tablet, a granule, powder, a capsule, or the like by a conventional method. These additives may be any additive that is commonly used in the field, and examples thereof include excipients such as lactose, white sugar, sodium chloride, glucose, starch, calcium carbonate, kaolin, microcrystalline cellulose, and silicic acid; binders such as water, ethanol, propanol, simple syrup, a glucose solution, a starch solution, a gelatin solution, carboxymethylcellulose, hydroxypropylcellulose, hydroxypropyl starch, methylcellulose, ethylcellulose, shellac, calcium phosphate, and polyvinylpyrrolidone; disintegrators such as dried starch, sodium alginate, agar powder, sodium hydrogencarbonate, calcium carbonate, sodium lauryl sulfate, monoglyceride stearate, and lactose; lubricants such as purified talc, a stearate, borax, and polyethylene glycol; and corrigents such as white sugar, orange peel, citric acid, and tartaric acid.

A liquid preparation for oral administration can be prepared by adding a corrigent, a buffer, a stabilizer, a flavoring agent, or the like, to a compound (1) of the present invention, and then forming the mixture into an internal liquid preparation, a syrup, an elixir, or the like by a conventional method. In this case, examples of additives include the above-mentioned corrigents; buffers such as sodium citrate; and stabilizers such as tragacanth, gum arabic, and gelatin.

An injection can be prepared by adding a pH adjuster, a buffer, a stabilizer, a tonicity, a topical anesthetic, or the like, to a compound (1) of the present invention, and then forming the mixture into subcutaneous, intramuscular, or intravenous injection by a conventional method. In this case, examples of the pH adjuster and the buffer include sodium citrate, sodium acetate, and sodium phosphate. Examples of the stabilizer include sodium pyrosulfite, EDTA, thioglycolic acid, and thiolactic acid. Examples of the topical anesthetic include procaine hydrochloride and lidocaine hydrochloride. Examples of the tonicity include sodium chloride and glucose.

A suppository can be prepared by adding a pharmaceutical carrier that is known in this field and, according to need, a surfactant, to a compound (1) of the present invention, and then forming the mixture into a suppository by a conventional method. Examples of the carrier include polyethylene glycol, lanolin, cacao butter, and fatty acid triglyceride. Examples of the surfactant include Tween^{™}.

An ointment can be prepared by blending, according to need, a base, a stabilizer, a humectant, a preservative, or the like that is commmonly used, to a compound (1) of the present invention, and then mixing them by a conventional method. Examples of the base include liquid paraffin, white Vaseline, white beeswax, octyldodecyl alcohol, and paraffin. Examples of the preservative include methyl p-hydroxybenzoate, ethyl p-hydroxybenzoate, and propyl p-hydroxybenzoate.

In addition to the above, the compound (1) of the present invention can be formed into a respiratory tonic, an ophthalmic solution, or a collunarium by a conventional method.

The dosage of the drug of the present invention may vary depending on the age, weight, symptom, administration formulation, administration frequency, and the like. In general, the compound (1) of the present invention is preferably administered orally or parenterally to an adult in a daily amount of 1 to 1000 mg once or several times per day.

### Examples

The present invention will now be specifically described with reference to examples, but is not limited thereto.

### <Manufacturing Example 1>

### Synthesis of 2-(S)-4-[N-(tert-butoxycarbonyl)glycyl]-1-(4-fluoroisoquinoline-5-sulfonyl)-2-methylhomopiperazine:

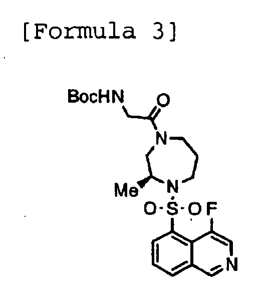

2-(S)-1-(4-Fluoroisoquinoline-5-sulfonyl)-2-methylhomopiperaz ine (396 mg), N-(tert-butoxycarbonyl)glycine (210 mg), and triethylamine (335 µL) were dissolved in chloroform (5 mL), and N-ethyl-N'-(3-dimethylaminopropyl)carbodiimide hydrochloride (230 mg) was added thereto under ice-cooling. The mixture was stirred at 0°C for 1 hour and subsequently at room temperature overnight. Water was added to the mixture to isolate the organic layer. The aqueous layer was extracted with chloroform. The organic layers were combined, washed with saturated brine, and dried over anhydrous magnesium sulfate. After concentration under reduced pressure, the residue was purified by silica gel column chromatography (solvent: ethyl acetate) to give the title compound as a colorless oil.
Yield: 445 mg (96%).

### <Manufacturing Example 2>

### Synthesis of 2-(S)-4-[N-(tert-butoxycarbonyl)-N-methylglycyl]-1-(4-fluoroisoqui noline-5-sulfonyl)-2-methylhomopiperazine:

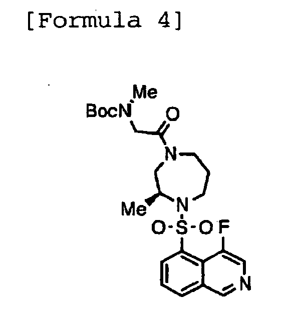

A reaction similar to that in Manufacturing Example 1 was carried out using
2-(S)-1-(4-fluoroisoquinoline-5-sulfonyl)-2-methylhomopiperazine (396 mg) and N-(tert-butoxycarbonyl)-N-(methyl)glycine (245 mg) to give the title compound as a colorless oil.
Yield: 393 mg (80%).

### <Manufacturing Example 3>

### Synthesis of 2-(S)-4-[N-(tert-butoxycarbonyl)-L-alanyl]-1-(4-fluoroisoquinoline -5-sulfonyl)-2-methylhomopiperazine:

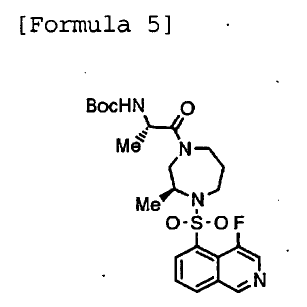

A reaction similar to that in Manufacturing Example 1 was carried out using
2-(S)-1-(4-fluoroisoquinoline-5-sulfonyl)-2-methylhomopiperazine (80 mg) and N- (tert-butoxycarbonyl)-L-alanine (46 mg) to give the title compound as a colorless oil.
Yield: 75 mg (63%).

### <Manufacturing Example 4>

### Synthesis of 2-(S)-4-[N-(tert-butoxycarbonyl)-D-alanyl]-1-(4-fluoroisoquinoline -5-sulfonyl)-2-methylhomopiperazine:

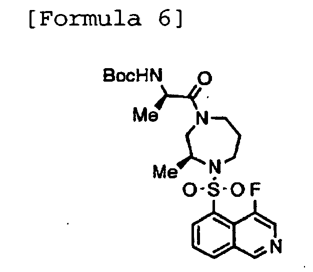

A reaction similar to that in Manufacturing Example 1 was carried out using
2-(S)-1-(4-fluoroisoquinoline-5-sulfonyl)-2-methylhomopiperazine (80 mg) and N- (tert-butoxycarbonyl)-D-alanine (46 mg) to give the title compound as a colorless oil.
Yield: 83 mg (69%).

### <Manufacturing Example 5>

### Synthesis of 2-(S)-4-[N-(tert-butoxycarbonyl)-L-valyl]-1-(4-fluoroisoquinoline-5-sulfonyl)-2-methylhomopiperazine:

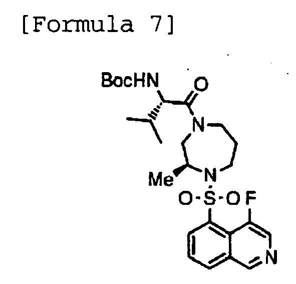

A reaction similar to that in Manufacturing Example 1 was carried out using
2-(S)-1-(4-fluoroisoquinoline-5-sulfonyl)-2-methylhomopiperazine (80 mg) and N- (tert-butoxycarbonyl)-L-valine (53 mg) to give the title compound as a colorless oil.
Yield: 83 mg (66%).

### <Manufacturing Example 6>

### Synthesis of 2-(S)-4-[N-(tert-butoxycarbonyl)-D-valyl]-1-(4-fluoroisoquinoline-5-sulfonyl)-2-methylhomopiperazine:

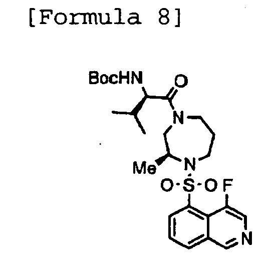

A reaction similar to that in Manufacturing Example 1 was carried out using
2-(S)-1-(4-fluoroisoquinoline-5-sulfonyl)-2-methylhomopiperazine (80 mg) and N- (tert-butoxycarbonyl) -D-valine (53 mg) to give the title compound as a colorless oil.
Yield: 92 mg (73%).

### <Manufacturing Example 7>

### Synthesis of 2-(S)-4-[N-(tert-butoxycarbonyl)-L-leucyl]-1-(4-fluoroisoquinoline -5-sulfonyl)-2-methylhomopiperazine:

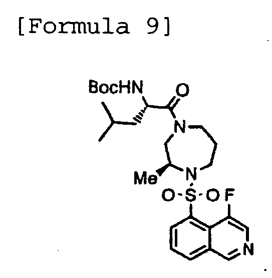

A reaction similar to that in Manufacturing Example 1 was carried out using
2-(S)-1-(4-fluoroisoquinoline-5-sulfonyl)-2-methylhomopiperazine (80 mg) and N- (tert-butoxycarbonyl) -L-leucine (56 mg) to give the title compound as a colorless oil.
Yield: 115 mg (89%).

### <Manufacturing Example 8>

### Synthesis of 2-(S)-4-[N-(tert-butoxycarbonyl)-D-leucyl]-1-(4-fluoroisoquinoline -5-sulfonyl)-2-methylhomopiperazine:

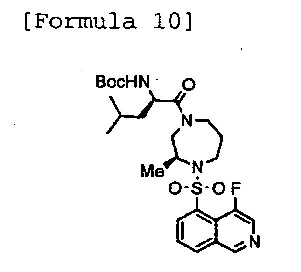

A reaction similar to that in Manufacturing Example 1 was carried out using
2-(S)-1-(4-fluoroisoquinoline-5-sulfonyl)-2-methylhomopiperazine (80 mg) and N-(tert-butoxycarbonyl)-D-leucine (56 mg) to give the title compound as a colorless oil.
Yield: 110 mg (85%).

### <Manufacturing Example 9>

### Synthesis of 2-(S)-4-[N-(tert-butoxycarbonyl)-β-alanyl]-1-(4-fluoroisoquinoline -5-sulfonyl)-2-methylhomopiperazine:

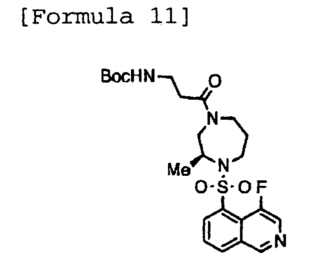

A reaction similar to that in Manufacturing Example 1 was carried out using
2-(S)-1-(4-fluoroisoquinoline-5-sulfonyl)-2-methylhomopiperazine (80 mg) and N- (tert-butoxycarbonyl)-β-alanine (46 mg) to give the title compound as a colorless oil.
Yield: 78 mg (65%).

### <Manufacturing Example 10>

### Synthesis of 2-(S)-4-[N-(tert-butoxycarbonyl)-1-aminocyclopropane-1-carbonyl]-1 -(4-fluoroisoquinoline-5-sulfonyl)-2-methylhomopiperazine:

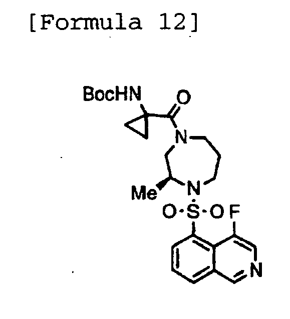

A reaction similar to that in Manufacturing Example 1 was carried out using
2-(S)-1-(4-fluoroisoquinoline-5-sulfonyl)-2-methylhomopiperazine (80 mg) and N-(tert-butoxycarbonyl)-1-aminocyclopropane-1-carboxylic acid (49 mg) to give the title compound as a colorless oil.
Yield: 71 mg (58%).

### <Manufacturing Example 11>

### Synthesis of 2-(S)-4-[N-(tert-butoxycarbonyl)-L-α-aminobutylyl]-1-(4-fluoroiso quinoline-5-sulfonyl)-2-methylhomopiperazine:

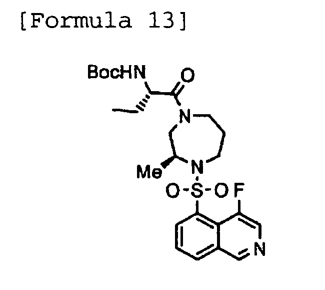

A reaction similar to that in Manufacturing Example 1 was carried out using
2-(S)-1-(4-fluoroisoquinoline-5-sulfonyl)-2-methylhomopiperazine (80 mg) and N-(tert-butoxycarbonyl)-L-α-aminobutyric acid (49 mg) to give the title compound as a colorless oil.
Yield: 63 mg (51%).

### <Manufacturing Example 12>

### Synthesis of 2-(S)-4-[N-(tert-butoxycarbonyl)-D-α-aminobutylyl]-1-(4-fluoroiso quinoline-5-sulfonyl)-2-methylhomopiperazine:

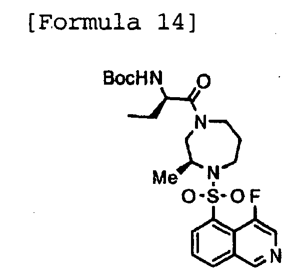

A reaction similar to that in Manufacturing Example 1 was carried out using
2-(S)-1-(4-fluoroisoquinoline-5-sulfonyl)-2-methylhomopiperazine (80 mg) and N- (tert-butoxycarbonyl)-D-α-aminobutyric acid (49 mg) to give the title compound as a colorless oil.
Yield: 88 mg (71%).

### <Manufacturing Example 13>

### Synthesis of 2-(S)-4-[N-(tert-butoxycarbonyl)-L-norleucyl]-1-(4-fluoroisoquinol ine-5-sulfonyl)-2-methylhomopiperazine:

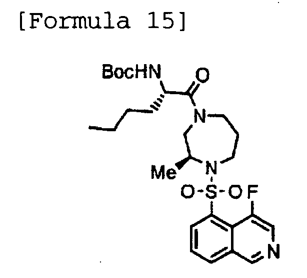

A reaction similar to that in Manufacturing Example 1 was carried out using
2-(S)-1-(4-fluoroisoquinoline-5-sulfonyl)-2-methylhomopiperazine (80 mg) and N-(tert-butoxycarbonyl)-L-norleucine (56 mg) to give the title compound as a colorless oil.
Yield: 97 mg (75%).

### <Manufacturing Example 14>

### Synthesis of 2-(S)-4-[N-(tert-butoxycarbonyl)-D-norleucyl]-1-(4-fluoroisoquinol ine-5-sulfonyl)-2-methylhomopiperazine:

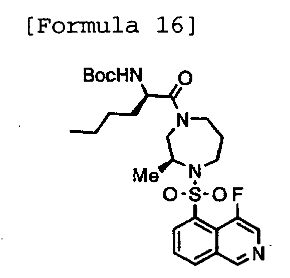

A reaction similar to that in Manufacturing Example 1 was carried out using
2-(S)-1-(4-fluoroisoquinoline-5-sulfonyl)-2-methylhomopiperazine (80 mg) and N-(tert-butoxycarbonyl)-D-norleucine (56 mg) to give the title compound as a colorless oil.
Yield: 112 mg (86%).

### <Manufacturing Example 15>

### Synthesis of 4-[N-(tert-butoxycarbonyl)glycyl]-1-(isoquinoline-5-sulfonyl)homop iperazine:

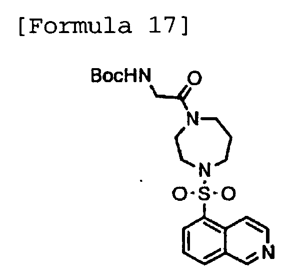

A reaction similar to that in Manufacturing Example 1 was carried out using 1-(isoquinoline-5-sulfonyl)homopiperazine (80 mg) and N-(tert-butoxycarbonyl)glycine (52 mg) to give the title compound as a colorless oil.
Yield: 120 mg (91%).

### <Manufacturing Example 16>

### Synthesis of 4-[N-(tert-butoxycarbonyl)-β-alanyl]-1-(isoquinoline-5-sulfonyl)ho mopiperazine:

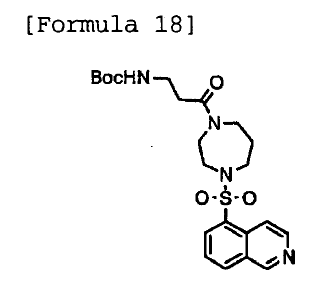

A reaction similar to that in Manufacturing Example 1 was carried out using 1-(isoquinoline-5-sulfonyl)homopiperazine (80 mg) and N-(tert-butoxycarbonyl)-β-alanine (55 mg) to give the title compound as a colorless oil.
Yield: 102 mg (75%).

### <Manufacturing Example 17>

### Synthesis of 4-[N-(tert-butoxycarbonyl)-L-alanyl]-1-(isoquinoline-5-sulfonyl)ho mopiperazine:

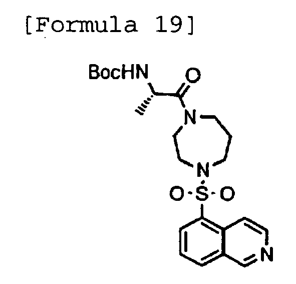

A reaction similar to that in Manufacturing Example 1 was carried out using 1-(isoquinoline-5-sulfonyl)homopiperazine (80 mg) and N-(tert-butoxycarbonyl)-L-alanine (55 mg) to give the title compound as a colorless oil.
Yield: 85 mg (63%).

### <Manufacturing Example 18>

### Synthesis of 4-[N-(tert-butoxycarbonyl)-D-alanyl]-1-(isoquinoline-5-sulfonyl)ho mopiperazine:

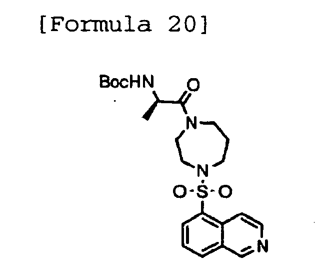

A reaction similar to that in Manufacturing Example 1 was carried out using 1-(isoquinoline-5-sulfonyl)homopiperazine (80 mg) and N-(tert-butoxycarbonyl)-D-alanine (55 mg) to give the title compound as a colorless oil.
Yield: 84 mg (62%).

### <Manufacturing Example 19>

### Synthesis of 4-[N-(tert-butoxycarbonyl)-L-valyl]-1-(isoquinoline-5-sulfonyl)hom opiperazine:

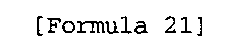

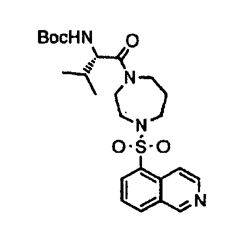

A reaction similar to that in Manufacturing Example 1 was carried out using 1-(isoquinoline-5-sulfonyl)homopiperazine (80 mg) and N-(tert-butoxycarbonyl)-L-valine (64 mg) to give the title compound as a colorless oil.
Yield: 60 mg (42%).

### <Manufacturing Example 20>

### Synthesis of 4-[N-(tert-butoxycarbonyl)-D-valyl]-1-(isoquinoline-5-sulfonyl)hom opiperazine:

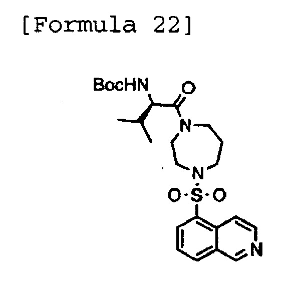

A reaction similar to that in Manufacturing Example 1 was carried out using 1-(isoquinoline-5-sulfonyl)homopiperazine (80 mg) and N-(tert-butoxycarbonyl)-D-valine (64 mg) to give the title compound as a colorless oil.
Yield: 68 mg (47%).

### <Manufacturing Example 21>

### Synthesis of 4-[N^{α},N^{β}-di-(tert-butoxycarbonyl)-L-α,β-diaminopropionyl]-1-(isoqu inoline-5-sulfonyl)homopiperazine:

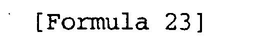

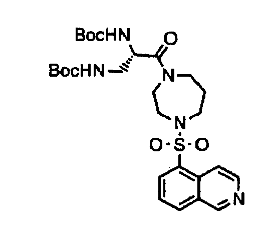

A reaction similar to that in Manufacturing Example 1 was carried out using 1-(isoquinoline-5-sulfonyl)homopiperazine (80 mg) and N^{α},N^{β}-di- (tert-butoxycarbonyl) -L-α,β-diaminopropionic acid (88 mg) to give the title compound as a colorless oil.
Yield: 59 mg (42%).

### <Manufacturing Example 22>

### Synthesis of 4-[N^{α},N^{β}-di-(tert-butoxycarbonyl)-D-α,β-diaminopropionyl]-1-(isoqu inoline-5-sulfonyl)homopiperazine:

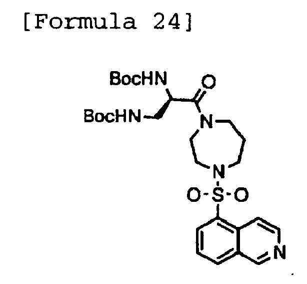

A reaction similar to that in Manufacturing Example 1 was carried out using 1-(isoquinoline-5-sulfonyl)homopiperazine (80 mg) and N^{α},N^{β}-di-(tert-butoxycarbonyl)-D-α,β-diaminopropionic acid (88 mg) to give the title compound as a colorless oil.
Yield: 59 mg (42%).

### <Manufacturing Example 23>

### Synthesis of 2-(S)-4-[N^{α},N^{β}-di-(tert-butoxycarbonyl)-L-α,β-diaminopropionyl]-1-(4-fluoroisoquinoline-5-sulfonyl)-2-methylhomopiperazine:

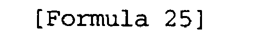

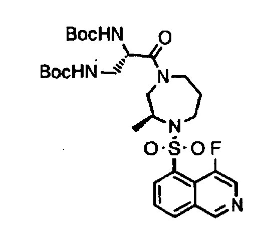

A reaction similar to that in Manufacturing Example 1 was carried out using
2-(S)-1-(4-fluoroisoquinoline-4-sulfonyl)-2-methylhomopiperazine (80 mg) and N^{α},N^{β}-di-(tert-butoxycarbonyl)-L-α,β-diaminopropionic acid (82 mg) to give the title compound as a colorless oil.
Yield: 42 mg (31%).

### <Manufacturing Example 24>

### Synthesis of 2-(S)-4-[N^{α},N^{β}-di-(tert-butoxycarbonyl)-D-α,β-diaminopropionyl]-1-(4-fluoroisoquinoline-5-sulfonyl)-2-methylhomopiperazine:

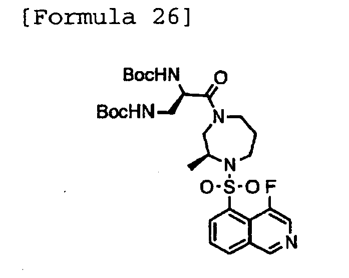

A reaction similar to that in Manufacturing Example 1 was carried out using
2-(S)-1-(4-fluoroisoquinoline-4-sulfonyl)-2-methylhomopiperazine (80 mg) and N^{α},N^{β}-di-(tert-butoxycarbonyl)-D-α,β-diaminopropionic acid (82 mg) to give the title compound as a colorless oil.
Yield: 34 mg (25%).

### <Manufacturing Example 25>

### Synthesis of 4-[N-(tert-butoxycarbonyl)glycyl]-1-(4-methylisoquinoline-5-sulfon yl)homopiperazine:

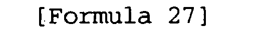

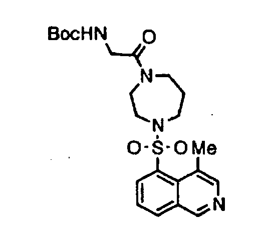

A reaction similar to that in Manufacturing Example 1 was carried out using 1-(4-methylisoquinoline-4-sulfonyl)homopiperazine (40 mg) and N- (tert-butoxycarbonyl) glycine (22 mg) to give the title compound as a colorless oil.
Yield: 49 mg (84%).

### <Manufacturing Example 26>

### Synthesis of 2-(S)-4-[N-(tert-butoxycarbonyl)glycyl]-1-(4-methylisoquinoline-5-sulfonyl)-2-methylhomopiperazine:

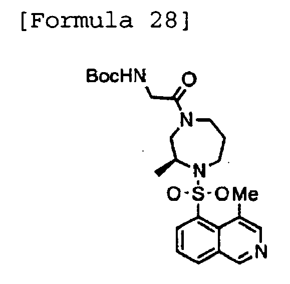

A reaction similar to that in Manufacturing Example 1 was carried out using
2-(S)-1-(4-methylisoquinoline-4-sulfonyl)-2-methylhomopiperazine (40 mg) and N-(tert-butoxycarbonyl)glycine (22 mg) to give the title compound as a colorless oil.
Yield: 46 mg (79%).

### <Manufacturing Example 27>

### Synthesis of 2-(S)-1-(4-bromoisoquinoline-5-sulfonyl)-4-[N-(tert-butoxycarbonyl )glycyl]-2-methylhomopiperazine:

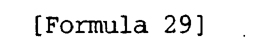

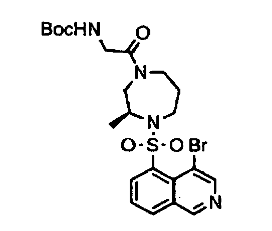

A reaction similar to that in Manufacturing Example 1 was carried out using
2-(S)-1-(4-methylisoquinoline-4-sulfonyl)-2-methylhomopiperazine (40 mg) and N-(tert-butoxycarbonyl)glycine (19 mg) to give the title compound as a colorless oil.
Yield: 40 mg (70%).

### <Manufacturing Example 28>

### Synthesis of 2-(S)-1-(4-ethenylisoquinoline-5-sulfonyl)-4-[N-(tert-butoxycarbon yl)glycyl]-2-methylhomopiperazine:

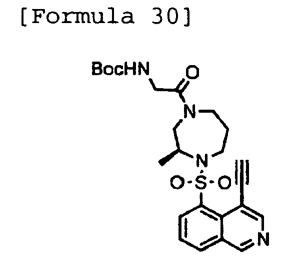

A reaction similar to that in Manufacturing Example 1 was carried out using
2-(S)-1-(4-ethenylisoquinoline-4-sulfonyl)-2-methylhomopiperazine (20 mg) and N-(tert-butoxycarbonyl)glycine (11 mg) to give the title compound as a colorless oil.
Yield: 9.7 mg (33%).

### <Example 1>

### Synthesis of 2-(S)-1-(4-fluoroisoquinoline-5-sulfonyl)-4-glycyl-2-methylhomopip erazine dihydrochloride:

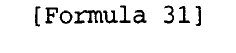

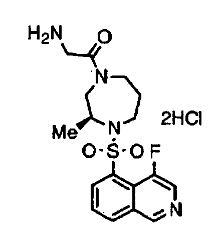

2-(S)-1-[N-(tert-Butoxycarbonyl)glycyl]-1-(4-fluoroisoquinoli ne-5-sulfonyl)-2-methylhomopiperazine (445 mg) was dissolved in methanol (2 mL), and an ethyl acetate solution (1 mL) of 4 M hydrogen chloride was added thereto. The mixture was stirred at room temperature for 4 hours and then concentrated under reduced pressure. The resulting precipitate was collected by filtration and washed with ethyl acetate to give the title compound as a white powder.
Yield: 347 mg (81%).
¹H-NMR (270 MHz, DMSO-d₆, 100°C) δ: 0.95 (d, 3H, J = 6.5 Hz), 1.78 (br. s, 2H), 3.36-3.50 (m, 3H), 3.70-3.79 (m, 3H), 4.18-4.25 (m, 1H), 7.90 (t, 1H, J = 7.8 Hz), 8.49-8.53 (m, 2H), 8.64 (d, 1H, J = 4.9 Hz), 9.31 (s, 1H).

### <Example 2>

### Synthesis of 2-(S)-1-(4-fluoroisoquinoline-5-sulfonyl)-2-methyl-4-(N-methylglyc yl)homopiperazine dihydrochloride:

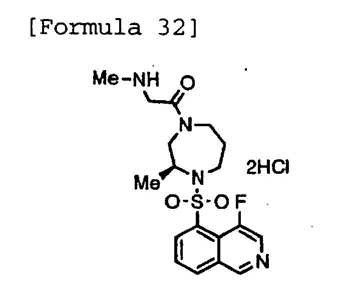

2-(S)-4-[N-(tert-Butoxycarbonyl)-N-methylglycyl]-1-(4-fluoroi soquinoline-5-sulfonyl)-2-methylhomopiperazine (393 mg) was treated as in Example 1 to give the title compound as a white powder.
Yield: 365 mg (98%).
¹H-NMR (270 MHz, DMSO-d₆, 100°C) δ: 0.92 (d, 3H, J = 6.8 Hz), 1.82 (br. s, 2H), 2.64 (s, 3H), 3.37-3.50 (m, 3H), 3.73-3.86 (m, 3H), 4.15-4.25 (m, 1H), 7.90 (t, 1H, J = 7.6 Hz), 8.50-8.53 (m, 2H), 8.65 (d, 1H, J = 5.4 Hz), 9.32 (s, 1H).

### <Example 3>

### Synthesis of 2-(S)-4-(L-alanyl)-1-(4-fluoroisoquinoline-5-sulfonyl)-2-methylhom opiperazine dihydrochloride:

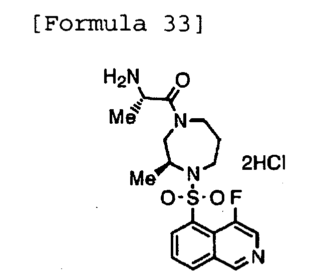

2-(S)-4-[N-(tert-Butoxycarbonyl)-L-alanyl]-1-(4-fluoroisoquin oline-5-sulfonyl)-2-methylhomopiperazine (75 mg) was treated as in Example 1 to give the title compound as a white powder.
Yield: 65 mg (92%).
¹H-NMR (270 MHz, DMSO-d₆, 100°C) δ: 0.96 (br. s, 3H), 1.44 (d, 3H, J = 7.0 Hz), 1.86 (br. s, 2H), 3.35-3.78 (m, 6H), 4.20-4.32 (m, 2H), 7.89 (t, 1H, J = 7.8 Hz), 8.45-8.51 (m, 2H), 8.62 (d, 1H, J = 5.4 Hz), 9.31 (s, 1H).

### <Example 4>

### Synthesis of 2-(S)-4-(D-alanyl)-1-(4-fluoroisoquinoline-5-sulfonyl)-2-methylhom opiperazine dihydrochloride:

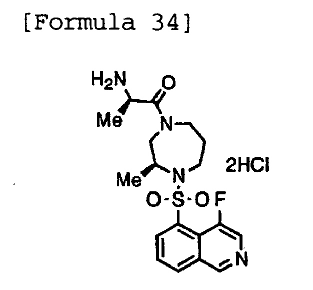

2-(S)-4-[N-(tert-Butoxycarbonyl)-D-alanyl]-1-(4-fluoroisoquin oline-5-sulfonyl)-2-methylhomopiperazine (83 mg) was treated as in Example 1 to give the title compound as a white powder.
Yield: 68 mg (87%).
¹H-NMR (270 MHz, DMSO-d₆, 100°C) δ: 0.95 (d, 3H, J = 6.5 Hz), 1.40 (d, 3H, J = 6.5 Hz), 1.86 (br. s, 2H), 3.43-3.55 (m, 3H), 3.77-3.83 (m, 3H), 4.15-4.35 (m, 2H), 7.89 (t, 1H, J = 8.1 Hz), 8.48-8.51 (m, 2H), 8.61 (d, 1H, J = 5.1 Hz), 9.31 (s, 1H).

### <Example 5>

### Synthesis of 2-(S)-1-(4-fluoroisoquinoline-5-sulfonyl)-2-methyl-4-(L-valyl)homo piperazine dihydrochloride:

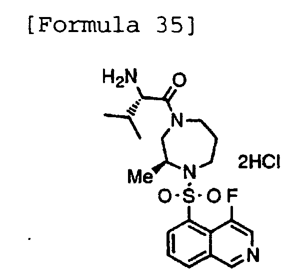

2-(S)-4-[N₋(tert-Butoxycarbonyl)-L-valyl]-1-(4-fluoroisoquino line-5-sulfonyl)-2-methylhomopiperazine (83 mg) was treated as in Example 1 to give the title compound as a white powder.
Yield: 61 mg (78%).
¹H-NMR (270 MHz, DMSO-d₆, 100°C) δ: 0.98 (d, 3H, J = 7.0 Hz), 1.05 (d, 6H, J = 6.8 Hz), 1.90 (br. s, 2H), 2.11-2.23 (m, 1H), 3.37-3.78 (m, 6H), 4.13 (br, 2H), 7.90 (t, 1H, J = 8.1 Hz), 8.44-8.52 (m, 2H), 8.61 (d, 1H, J = 4.9 Hz), 9.31 (s, 1H).

### <Example 6>

### Synthesis of 2-(S)-1-(4-fluoroisoquinoline-5-sulfonyl)-2-methyl-4-(D-valyl)homo piperazine dihydrochloride:

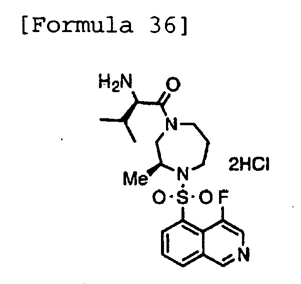

2-(S)-4-[N-(tert-Butoxycarbonyl)-D-valyl]-1-(4-fluoroisoquino line-5-sulfonyl)-2-methylhomopiperazine (92 mg) was treated as in Example 1 to give the title compound as a white powder.
Yield: 79 mg (91%).
¹H-NMR (270 MHz, DMSO-d₆, 100°C) δ: 1.01 (d, 3H, J = 6.7 Hz), 1.05 (d, 6H, J = 6.8 Hz), 1.90 (br. s, 2H), 2.13-2.19 (m, 1H), 3.59-3.69 (m, 6H), 4.16 (br, 2H), 7.89 (t, 1H, J = 7.6 Hz), 8.49-8.52 (m, 2H), 8.62 (d, 1H, J = 4.9 Hz), 9.31 (s, 1H).

### <Example 7>

### Synthesis of 2-(S)-1-(4-fluoroisoquinoline-5-sulfonyl)-4-(L-leucyl)-2-methylhom opiperazine dihydrochloride:

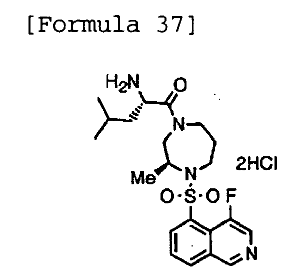

2-(S)-4-[N-(tert-Butoxycarbonyl)-L-leucyl]-1-(4-fluoroisoquin oline-5-sulfonyl)-2-methylhomopiperazine (115 mg) was treated as in Example 1 to give the title compound as a white powder.
Yield: 97 mg (89%).
¹H-NMR (270 MHz, DMSO-d₆, 100°C) δ: 0.93 (d, 6H, J = 6.8 Hz), 0.96 (d, 3H, J = 6.5 Hz), 1.15-1.92 (m, 3H), 3.43-3.75 (m, 6H), 4.19-4.27 (m, 2H), 7.90 (t, 1H, J = 8.1 Hz), 8.45-8.53 (m, 2H), 8.62 (d, 1H, J = 5.4 Hz), 9.31 (s, 1H).

### <Example 8>

### Synthesis of 2-(S)-1-(4-fluoroisoquinoline-5-sulfonyl)-4-(D-leucyl)-2-methylhom opiperazine dihydrochloride:

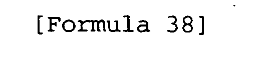

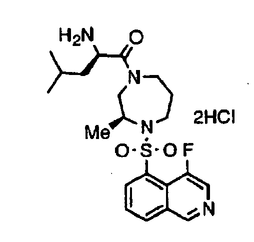

2-(S)-4-[N-(tert-Butoxycarbonyl)-D-leucyl]-1-(4-fluoroisoquin oline-5-sulfonyl)-2-methylhomopiperazine (110 mg) was treated as in Example 1 to give the title compound as a white powder.
Yield: 95 mg (91%).
¹H-NMR (270 MHz, DMSO-d₆, 100°C) δ: 0.91 (d, 6H, J = 6.5 Hz), 0.96 (d, 3H, J = 6.5 Hz), 1.57-1.92 (m, 3H), 3.45-3.58 (m, 3H), 3.73-3.81 (m, 3H), 4.16-4.25 (m, 2H), 7.89 (t, 1H, J = 8.1 Hz), 8.48-8.52 (m, 2H), 8.61 (d, 1H, J = 5.1 Hz), 9.31 (s, 1H).

### <Example 9>

### Synthesis of 2-(S)-4-(β-alanyl)-1-(4-fluoroisoquinoline-5-sulfonyl)-2-methylhom opiperazine dihydrochloride:

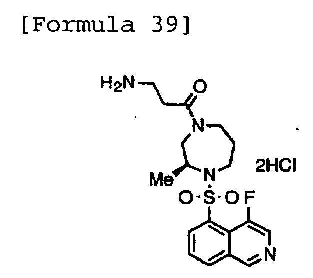

2-(S)-4-[N-(tert-Butoxycarbonyl)-β-alanyl]-1-(4-fluoroisoquin oline-5-sulfonyl)-2-methylhomopiperazine (78 mg) was treated as in Example 1 to give the title compound as a white powder.
Yield: 64 mg (87%).
¹H-NMR (270 MHz, DMSO-d₆, 100°C) δ: 0.88 (br. s, 3H), 1.78 (br, 2H), 2.81 (br, 2H), 3.07 (br, 2H), 3.25-3.50 (m, 3H), 3.76-4.01 (m, 3H), 4.11-4.24 (m, 1H), 7.89 (t, 1H, J = 8.1 Hz), 8.49-8.53 (m, 2H), 8.61 (d, 1H, J = 4.9 Hz), 9.31.

### <Example 10>

### Synthesis of 2-(S)-4-(1-aminocyclopropane-1-carbonyl)-1-(4-fluoroisoquinoline-5 -sulfonyl)-2-methylhomopiperazine dihydrochloride:

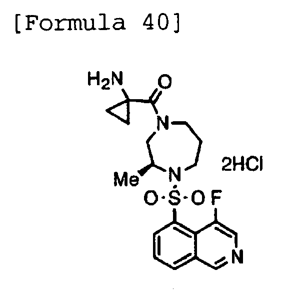

2-(S)-4-[N-(tert-Butoxycarbonyl)-1-aminocyclopropane-1-carbon yl]-1-(4-fluoroisoquinoline-5-sulfonyl)-2-methylhomopiperazine (71 mg) was treated as in Example 1 to give the title compound as a white powder.
Yield: 60 mg (89%).
¹H-NMR (270 MHz, DMSO-d₆, 100°C) δ: 0.88 (d, 3H, J = 6.5 Hz), 1.16-1.23 (m, 2H), 1.33-1.47 (m, 2H), 1.81-1. 84 (m, 2H), 3.27-3.53 (m, 3H), 3.75-3.84 (m, 1H), 3.92-4.06 (m, 2H), 4.11-4.21 (m, 1H), 7.89 (t, 1H, J = 7.6 Hz), 8.46-8.52 (m, 2H), 8.61 (d, 1H, J = 4.7 Hz), 9.31 (s, 1H) .

### <Example 11>

### Synthesis of 2-(S)-4-(L-α-aminobutylyl)-1-(4-fluoroisoquinoline-5-sulfonyl)-2-methylhomopiperazine dihydrochloride:

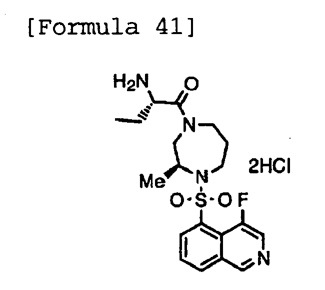

2-(S)-4-[N-(tert-Butoxycarbonyl)-L-α-aminobutylyl]-1-(4-fluor oisoquinoline-5-sulfonyl)-2-methylhomopiperazine (63 mg) was treated as in Example 1 to give the title compound as a white powder.
Yield: 48 mg (82%).
¹H-NMR (270 MHz, DMSO-d₆, 100°C) δ: 0.95-1.00 (m, 6H), 1.75-1.92 (m, 4H), 3.40-3.76 (m, 6H), 4.19-4.24 (m, 2H), 7.90 (t, 1H, J = 8.1 Hz), 8.44-8.49 (m, 2H), 8.62 (d, 1H, J = 5.4 Hz), 9.31 (s, 1H).

### <Example 12>

### Synthesis of 2-(S)-4-(D-α-aminobutylyl)-1-(4-fluoroisoquinoline-5-sulfonyl)-2-methylhomopiperazine dihydrochloride:

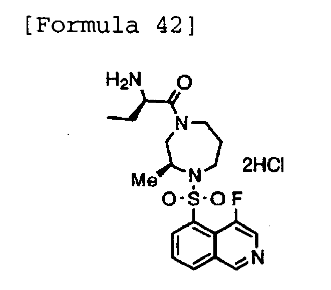

2-(S)-4-[N-(tert-Butoxycarbonyl)-D-α-aminobutylyl]-1-(4-fluor oisoquinoline-5-sulfonyl)-2-methylhomopiperazine (88 mg) was treated as in Example 1 to give the title compound as a white powder.
Yield: 70 mg (84%).
¹H-NMR (270 MHz, DMSO-d₆, 100°C) δ: 0.93-1.01 (m, 6H), 1.79-1.92 (m, 4H), 3.49-3.81 (m, 6H), 4.17-4.27 (m, 2H), 7.89 (t, 1H, J = 7.8 Hz), 8.48-8.52 (m, 2H), 8.61 (d, 1H, J = 5.1 Hz), 9.31 (s, 1H).

### <Example 13>

### Synthesis of 2-(S)-1-(4-fluoroisoquinoline-5-sulfonyl)-2-methyl-4-(L-norleucyl) homopiperazine dihydrochloride:

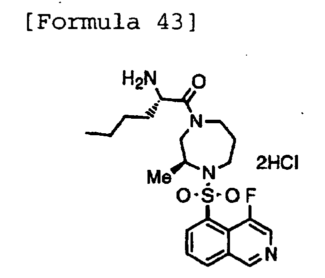

2-(S)-4-[N-(tert-Butoxycarbonyl)-L-norleucyl]-1-(4-fluoroisoq uinoline-5-sulfonyl)-2-methylhomopiperazine (87 mg) was treated as in Example 1 to give the title compound as a white powder.
Yield: 75 mg (81%).
¹H-NMR (270 MHz, DMSO-d₆, 100°C) δ: 0.86-0.99 (m, 6H), 1.13-1.47 (m, 4H), 1. 69-1.19 (m, 4H), 3.43-3.77 (m, 6H), 4.21-4.24 (m, 2H), 7.90 (t, 1H, J = 7.8 Hz), 8.44-8.52 (m, 2H), 8.63 (d, 1H, J = 4.9 Hz), 9.31 (s, 1H) .

### <Example 14>

### Synthesis of 2-(S)-1-(4-fluoroisoquinoline-5-sulfonyl)-2-methyl-4-(D-norleucyl) homopiperazine dihydrochloride:

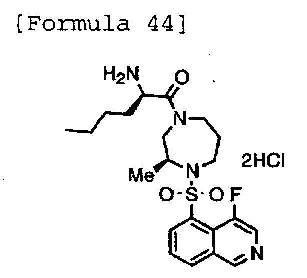

2-(S)-4-[N-(tert-Butoxycarbonyl)-D-norleucyl]-1-(4-fluoroisoq uinoline-5-sulfonyl)-2-methylhomopiperazine (112 mg) was treated as in Example 1 to give the title compound as a white powder.
Yield: 96 mg (90%).
¹H-NMR (270 MHz, DMSO-d₆, 100°C) δ: 0.89 (t, 3H, J = 7.3 Hz), 0.95 (d, 3H, J = 6.2 Hz), 1.29-1.42 (m, 4H), 1.74-1.93 (m, 4H), 3.45-3.53 (m, 3H), 3.75-3.83 (m, 3H), 4.21-4.28 (m, 2H), 7.89 (t, 1H, J = 7.8 Hz), 8.49-8.52 (m, 2H), 8.61 (d, 1H, J = 5.1 Hz), 9.31 (s, 1H).

### <Example 15>

### Synthesis of 4-(glycyl)-1-(isoquinoline-5-sulfonyl)homopiperazine dihydrochloride:

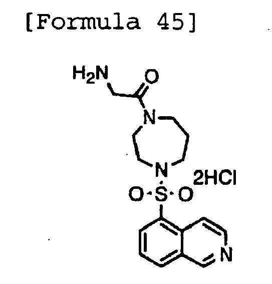

4-[N-(tert-Butoxycarbonyl)glycyl]-1-(isoquinoline-5-sulfonyl) homopiperazine (120 mg) was treated as in Example 1 to give the title compound as a white powder.
Yield: 82 mg (73%).
¹H-NMR (270 MHz, DMSO-d₆, 100°C) δ: 1.85 (br, 2H), 3.42-3.61 (m, 8H), 3.78 (s, 2H), 7.89 (t, 1H, J = 8.4 Hz), 8.39 (dd, 1H, J = 7.3 Hz, 1.1 Hz), 8.45-8.52 (m, 2H), 8.71 (d, 1H, J = 6.2 Hz), 9.57 (s, 1H).

### <Example 16>

### Synthesis of 4-(β-alanyl)-1-(isoquinoline-5-sulfonyl)homopiperazine dihydrochloride:

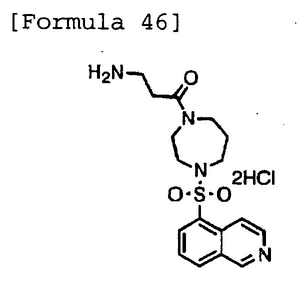

4-[N-(tert-Butoxycarbonyl)-β-alanyl]-1-(isoquinoline-5-sulfon yl)homopiperazine (102 mg) was treated as in Example 1 to give the title compound as a white powder.
Yield: 67 mg (70%).
¹H-NMR (270 MHz, DMSO-d₆, 100°C) δ: 1.82 (br, 2H), 2.74 (t, 2H, J = 6.8 Hz), 3.44-3.62 (m, 10H), 8.41 (dd, 1H, J = 5.7 Hz, 1.1 Hz), 8.47-8.53 (m, 2H), 8.71 (d, 1H, J = 6.2 Hz), 9.58 (s, 1H).

### <Example 17>

### Synthesis of 4-(L-alanyl)-1-(isoquinoline-5-sulfonyl)homopiperazine dihydrochloride:

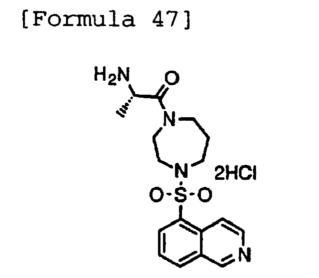

4-[N-(tert-Butoxycarbonyl)-L-alanyl]-1-(isoquinoline-5-sulfon yl)homopiperazine (85 mg) was treated as in Example 1 to give the title compound as a white powder.
Yield: 54 mg (68%).
¹H-NMR (270 MHz, DMSO-d₆, 100°C) δ: 1.35 (d, 3H, J = 7.0 Hz), 1.87 (br, 2H), 3.41-3.62 (m, 8H), 4.23 (q, 1H, J = 7.0 Hz), 7.85 (t, 1H, J = 7.8 Hz), 8.36 (dd, 1H, J = 7.6 Hz, 1.4 Hz), 8.42 (d, 1H, J = 5.9 Hz), 8.46 (d, 1H, J = 8.3 Hz), 8.69 (d, 1H, J = 5.9 Hz), 9.51 (s, 1H).

### <Example 18>

### Synthesis of 4-(D-alanyl)-1-(isoquinoline-5-sulfonyl)homopiperazine dihydrochloride:

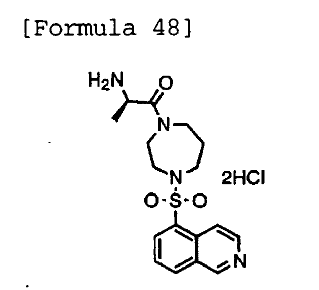

4-[N-(tert-Butoxycarbonyl)-D-alanyl]-1-(isoquinoline-5-sulfon yl)homopiperazine (84 mg) was treated as in Example 1 to give the title compound as a white powder.
Yield: 57 mg (72%).
¹H-NMR (270 MHz, DMSO-d₆, 100°C) δ: 1.36 (d, 3H, J = 6.8 Hz), 1.87 (br, 2H), 3.49-3.61 (m, 8H), 4.23 (q, 1H, J = 7.0 Hz), 7.85 (t, 1H, J = 7.8 Hz), 8.37 (dd, 1H, J = 7.0 Hz, 0.8 Hz), 8.42 (d, 1H, J = 6.2 Hz), 8.46 (d, 1H, J = 8.4 Hz), 8.69 (d, 1H, J = 6.2 Hz), 9.51 (s, 1H).

### <Example 19>

### Synthesis of 1-(isoquinoline-5-sulfonyl)-4-(L-valyl)homopiperazine dihydrochloride:

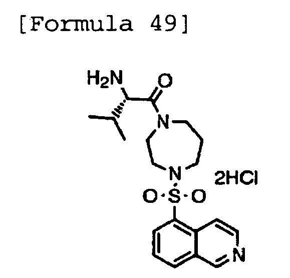

4-[N-(tert-Butoxycarbonyl)-L-valyl]-1-(isoquinoline-5-sulfony 1) homopiperazine (60 mg) was treated as in Example 1 to give the title compound as a white powder.
Yield: 44 mg (78%).
¹H-NMR (270 MHz, DMSO-d₆, 100°C) δ: 0.95 (d, 3H, J = 7.0 Hz), 0.99 (d, 3H, J = 7.0 Hz), 1.90 (br, 2H), 2.00-2.15 (m, 1H), 3.52-3.79 (m, 8H), 4.07 (d, 1H, J = 5.4 Hz), 7.85 (t, 1H, J = 7.8 Hz), 8.37 (dd, 1H, J = 7.3 Hz, 1.1 Hz), 8.42-8.48 (m, 2H), 8.69 (d, 1H, J = 6.2 Hz), 9.51 (s, 1H).

### <Example 20>

### Synthesis of 1-(isoquinoline-5-sulfonyl)-4-(D-valyl)homopiperazine dihydrochloride:

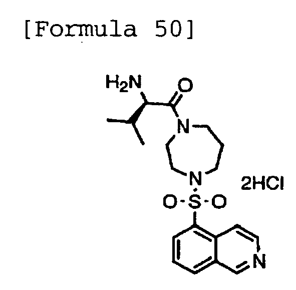

4-[N-(tert-Butoxycarbonyl)-D-valyl]-1-(isoquinoline-5-sulfony 1)homopiperazine (68 mg) was treated as in Example 1 to give the title compound as a white powder.
Yield: 43 mg (67%).
¹H-NMR (270 MHz, DMSO-d₆, 100°C) δ: 0.95 (d, 3H, J = 7.0 Hz), 0.99 (d, 3H, J = 7.0 Hz), 1.90 (br, 2H), 2.03-2.13 (m, 1H), 3.53-3.79 (m, 8H), 4.07 (d, 1H, J = 5.7 Hz), 7.87 (t, 1H, J = 7.6 Hz), 8.39 (d, 1H, J = 7.6 Hz), 8.45-8.50 (m, 2H), 8.70 (d, 1H, J = 6.2 Hz), 9.53 (s, 1H).

### <Example 21>

### Synthesis of 4-(L-α,β-diaminopropionyl)-1-(isoquinoline-5-sulfonyl)homopiperazi ne trihydrochloride:

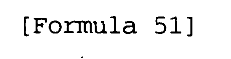

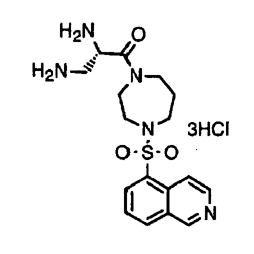

4-[N^{α},N^{β}-Di-(tert-butoxycarbonyl)-L-α,β-diaminopropionyl]-1-(i soquinoline-5-sulfonyl)homopiperazine (59 mg) was treated as in Example 1 to give the title compound as a white powder.
Yield: 40 mg (80%).
¹H-NMR (270 MHz, DMSO-d₆, 100°C) δ: 1.91 (br, 2H), 3.21-3.75 (m, 10H), 4.67-4.71 (m, 1H), 7.86 (t, 1H, J = 8.1 Hz), 8.37-8.48 (m, 3H), 8.70 (d, 1H, J = 6.2 Hz), 9.51 (s, 1H).

### <Example 22>

### Synthesis of 4-(D-α,β-diaminopropionyl)-1-(isoquinoline-5-sulfonyl)homopiperazi ne trihydrochloride:

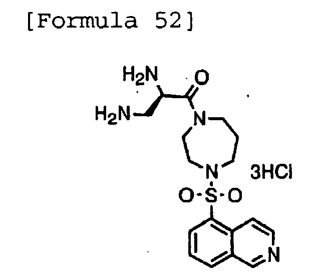

4-[N^{α},N^{β}-Di-(tert-butoxycarbonyl)-D-α,β-diaminopropionyl]-1-(i soquinoline-5-sulfonyl)homopiperazine (60 mg) was treated as in Example 1 to give the title compound as a white powder.
Yield: 40 mg (79%).
¹H-NMR (270 MHz, DMSO-d₆, 100°C) δ: 1.92 (br, 2H), 3.24-3.73 (m, 10H), 4.68-4.73 (m, 1H), 7.88 (t, 1H, J = 8.1 Hz), 8.39-8.51 (m, 3H), 8.71 (d, 1H, J = 5.9 Hz), 9.54 (s, 1H).

### <Example 23>

### Synthesis of 2-(S)-4-(L-α,β-diaminopropionyl)-1-(4-fluoroisoquinoline-5-sulfony 1)-2-methylhomopiperazine trihydrochloride:

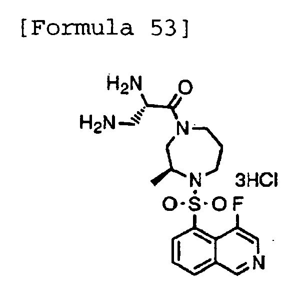

2-(S)-4-[N^{α},N^{β}-Di-(tert-butoxycarbonyl)-L-α,β-diaminopropionyl ]-1-(4-fluoroisoquinoline-5-sulfonyl)-2-methylhomopiperazine (42 mg) was treated as in Example 1 to give the title compound as a white powder.
Yield: 29 mg (81%).
¹H-NMR (270 MHz, DMSO-d₆, 100°C) δ: 0.92 (d, 3H, J = 6.8 Hz), 1.88 (br, 2H), 3.29-3.57 (m, 5H), 3.78-3.86 (m, 3H), 4.19-4.26 (m, 1H), 4.75-4.78 (m, 1H), 7.90 (t, 1H, J = 8.1 Hz), 8.49-8.53 (m, 2H), 8.62 (d, 1H, J = 5.1 Hz), 9.31 (s, 1H).

### <Example 24>

### Synthesis of 2-(S)-4-(D-α,β-diaminopropionyl)-1-(4-fluoroisoquinoline-5-sulfony 1)-2-methylhomopiperazine trihydrochloride:

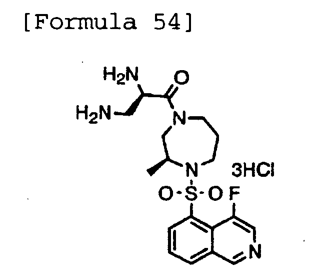

2-(S)-4-[N^{α},N^{β}-Di-(tert-butoxycarbonyl)-D-α,β-diaminopropionyl ]-1-(4-fluoroisoquinoline-5-sulfonyl)-2-methylhomopiperazine (34 mg) was treated as in Example 1 to give the title compound as a white powder.
Yield: 23 mg (80%).
¹H-NMR (270 MHz, DMSO-d₆, 100°C) δ: 0.86 (d, 3H, J = 6.8 Hz), 1.93 (br, 2H), 3.22-3.45 (m, 5H), 3.81-3.87 (m, 3H), 4.08-4.18 (m, 1H), 4.78 (br, 1H), 7.89 (t, 1H, J = 7.8 Hz), 8.50-8.53 (m, 2H), 8.62 (d, 1H, J = 5.1 Hz), 9.31 (s, 1H).

### <Example 25>

### Synthesis of 4-(glycyl)-1-(4-methylisoquinoline-5-sulfonyl)homopiperazine dihydrochloride:

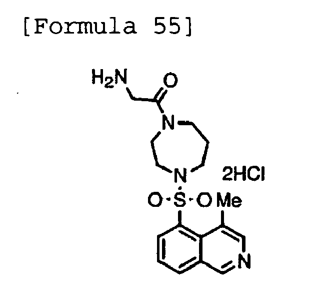

4-[N-(tert-Butoxycarbonyl)glycyl]-1-(4-methylisoquinoline-5-s ulfonyl) homopiperazine (49 mg) was treated as in Example 1 to give the title compound as a white powder.
Yield: 28 mg (61%).
¹H-NMR (270 MHz, DMSO-d₆, 100°C) δ: 1.93 (br, 2H), 2.97 (s, 3H), 3.58-3.69 (m, 8H), 3.86 (s, 2H), 7.77 (t, 1H, J = 7.6 Hz), 8.08 (d, 1H, J = 7.6 Hz), 8.39 (dd, 1H, J = 8.1 Hz, 1.1 Hz), 8.52 (d, 1H, J = 1.1 Hz), 9.29 (s, 1H).

### <Example 26>

### Synthesis of 2-(S)-4-(glycyl)-1-(4-methylisoquinoline-5-sulfonyl)-2-methylhomop iperazine dihydrochloride:

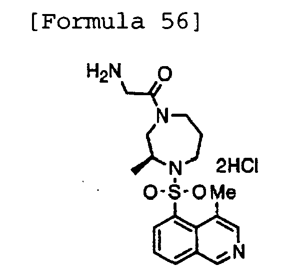

2-(S)-4-[N-(tert-Butoxycarbonyl)glycyl]-1-(4-methylisoquinoli ne-5-sulfonyl)-2-methylhomopiperazine (46 mg) was treated as in Example 1 to give the title compound as a white powder.
Yield: 34 mg (78%).
¹H-NMR (270 MHz, DMSO-d₆, 100°C) δ: 1.14 (d, 3H, J = 5.9 Hz), 1.83-2.01 (m, 2H), 2.96 (s, 3H), 3.42-3.71 (m, 7H), 3.87 (dd, 2H, J = 14.6 Hz, 5.1 Hz), 7.80 (t, 1H, J = 8.1 Hz), 8.27 (dd, 1H, J = 7.6 Hz, 1.1 Hz), 8.40 (dd, 1H, J = 7.8 Hz, 1.4 Hz), 8.52 (d, 1H, J = 0.5 Hz), 9.29 (s, 1H).

### <Example 27>

### Synthesis of 2-(S)-1-(4-bromoisoquinoline-5-sulfonyl)-4-(glycyl)-2-methylhomopi perazine dihydrochloride:

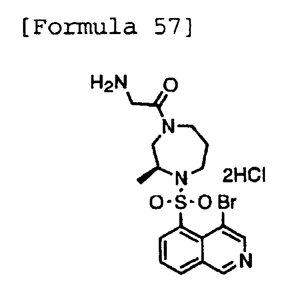

2-(S)-1-(4-Bromoisoquinoline-5-sulfonyl)-4-[N-(tert-butoxycar bonyl)glycyl]-2-methylhomopiperazine (40 mg) was treated as in Example 1 to give the title compound as a white powder.
Yield: 32 mg (84%).
¹H-NMR (270 MHz, DMSO-d₆, 100°C) δ: 1.06-1.10 (m, 3H), 1.86 (br, 2H), 3.42-3.55 (m, 3H), 3.68-3.88 (m, 3H), 4.18-4.24 (m, 3H), 7.86 (t, 1H, J = 7.6), 8.38 (dd, 1H, J = 7.6 Hz, 1.4 Hz), 8.45 (dd, 1H, J = 8.1 Hz, 1.4 Hz), 8.90 (s, 1H), 9.37 (s, 1H).

### <Example 28>

### Synthesis of 2-(S)-1-(4-ethenylisoquinoline-5-sulfonyl)-4-(glycyl)-2-methylhomo piperazine dihydrochloride:

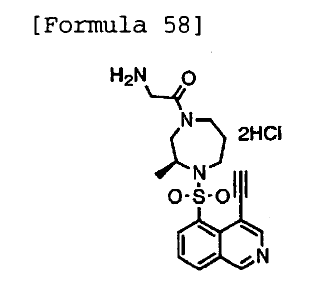

2-(S)-1-(4-Ethenylisoquinoline-5-sulfonyl)-4-[N-(tert-butoxyc arbonyl)glycyl]-2-methylhomopiperazine (9.7 mg) was treated as in Example 1 to give the title compound as a white powder.
Yield: 5.0 mg (55%).
M/Z: 386 [M⁺]

### Test Example 1: Measurement of kinase-inhibiting activity

### <ROCKII: Rho-associated coiled-coil forming kinase-II (Rho-kinase)>

An Rho-kinase assay was carried out according to the method described in Patent Document 1, and the concentration of a compound for inhibiting Rho-kinase activity by 50% (hereinafter referred to as "IC₅₀ value") was calculated.

### <PKA: Protein kinase A>

Purified bovine PKA (catalyst subunit, Promega)was used to evaluate its activity to phosphorylate a substrate peptide (LRRASLG, Promega).

In a system of the total volume of 50 µL, the reaction of the above-mentioned enzyme (43 ng) was carried out at room temperature for 20 minutes in the presence of 40 mM of Tris-HCl (pH 7.5), 20 mM of magnesium chloride, 1 mg/mL of BSA, 5 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <PKC: Protein kinase C>

Purified rat PKC (containing PKCα, β, and γ and small amounts of δ and ζ isoforms, Promega) was used to evaluate its activity to phosphorylate a substrate peptide (AAKIQASFRGHMARKK, Promega).

In a system of the total volume of 50 µL, the reaction of the above-mentioned enzyme (5 ng) was carried out at room temperature for 80 minutes in the presence of 20 mM of Tris-HCl (pH 7.5), 20 mM of magnesium chloride, 0.4 mM of calcium chloride, 0.1 mg/mL of BSA, 0.25 mM of EGTA, 0.5 mg/mL of phosphatidylserine (Sigma), 0.05 mg/mL of 1,2-dioctanoyl-sn-glycerol (Sigma), 10 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <PKG: Protein kinase G>

Purified bovine PKGα (Promega) was used to evaluate its activity to phosphorylate a substrate peptide (RKISASEF, Promega).

In a system of the total volume of 50 µL, the reaction of the above-mentioned enzyme (64 ng) was carried out at room temperature for 80 minutes in the presence of 40 mM of Tris-HCl (pH 7.5), 20 mM of magnesium chloride, 10 µM of cGMP (Sigma), 100 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <MLCK: Myosin light chain kinase>

Purified chicken MLCK (purified by the method of M. Anne Conti, et al. : Method in Enzymol. 196, 34-47) was used to evaluate its activity to phosphorylate a substrate peptide (AKRPQRATSNVFS, Sigma).

In a system of the total volume of 50 µL, the reaction of the above-mentioned enzyme (100 ng) was carried out at room temperature for 80 minutes in the presence of 20 mM of Tris-HCl (pH 7.5), 5 mM of magnesium chloride, 0.2 mM of calcium chloride, 0.02 mg/mL of BSA, 8 µg/mL of purified bovine brain calmodulin (Sigma), 50 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <CaMKII: Calcium/calmodulin-dependent protein kinase-II>

Purified rat CaMKII (containing CaMKIIα and small amounts of β, γ, and δ isoforms, Upstate) was used to evaluate its activity to phosphorylate a substrate peptide (KKALRRQETVDAL, Upstate).

In a system of the total volume of 50 µL, the reaction of the above-mentioned enzyme (33 ng) was carried out at room temperature for 60 minutes in the presence of 50 mM of Tris-HCl (pH 7.5), 10 mM of magnesium chloride, 2 mM of calcium chloride, 0.04 mg/mL of BSA, 16 µg/mL of purified bovine brain calmodulin (Sigma), 0.5 mM of DTT, 50 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <AuroraA: Aurora kinase A>

Recombinant human AuroraA (Upstate) was used to evaluate its activity to phosphorylate a substrate peptide (LRRASLG, Promega) was evaluated.

In a system of the total volume of 50 µL, the reaction of the above-mentioned enzyme (40 ng) was carried out at room temperature for 80 minutes in the presence of 40 mM of MOPS-NaOH (pH 7.5), 20 mM of magnesium chloride, 200 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <MSK1: Mitogen- and stress-activated protein kinase-1>

By using recombinant human MSK1 (from 2nd to 832nd amino acids, Upstate), activity of phosphorylating a substrate peptide (LRRASLG, Promega) was evaluated.

In a system of the total volume of 50 µL, the reaction of the above-mentioned enzyme (40 ng) was carried out at room temperature for 80 minutes in the presence of 40 mM of MOPS-NaOH (pH 7.5), 20 mM of magnesium chloride, 200 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <MKK4: Mitogen-activated protein kinase kinase 4>

Recombinant mouse MKK4 (from 34th to 397th amino acids, Upstate) was used to evaluate its activity to phosphorylate recombinant human JNK1α1 (inactive type, Upstate).

In a system of the total volume of 50 µL, the reaction of the above-mentioned enzyme (400 ng) was carried out at room temperature for 80 minutes in the presence of 25 mM of Tris-HCl (pH 7.5), 10 mM of magnesium chloride, 0.02 mg/mL of the above JNK1α1, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <Ab1: Oncogene product>

Recombinant human Ab1 (from 27th to 1149th amino acids, Upstate) was used to evaluate its activity to phosphorylate a substrate peptide (EAIYAAPFAKKK, Upstate).

In a system of the total volume of 50 µL, the reaction of the above-mentioned enzyme (20 ng) was carried out at room temperature for 80 minutes in the presence of 50 mM of Tris-HCl (pH 7.5), 10 mM of magnesium chloride, 50 µM of the above peptide, and 1 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

### <Src: Oncogene product>

Recombinant human Src (Upstate) was used to evaluate its activity to phosphorylate a substrate peptide (KVEKIGEGTYGVVYK, Upstate).

In a system of the total volume of 50 µL, the reaction of the above-mentioned enzyme (6 Units) was carried out at room temperature for 80 minutes in the presence of 40 mM of MOPS-NaOH (pH 7.5), 10 mM of magnesium chloride, 25 mM of manganese chloride, 300 mM of the above peptide, and 0.5 µM of ATP. After the kinase reaction, the luminescence intensity corresponding to the amount of ATP in the reaction system was measured using a luciferase reagent (Kinase-Glo Luminescent Kinase Assay kit, Promega) with a chemiluminescence measurement apparatus (MicroLumat, Berthold Technologies). The amount of ATP consumed by the phosphorylation reaction was calculated by comparison with the luminescence intensity in the system when the kinase reaction was not carried out.

With this system, the kinase reaction was carried out in the presence of a compound at various concentrations, and the IC₅₀ value of the compound was determined based on the resulting compound concentration-response curve for ATP consumption.

Table 1 shows inhibitory activities of the compound 4 disclosed in Patent Document 1 and the compounds in the above Examples against each kinase mentioned above.

### Abbreviations

BSA: bovine serum albumin
EGTA: glycoletherdiaminetetraacetic acid
cGMP: cyclic guanosine monophosphate
MOPS: 3-morpholinopropanesulfonic acid

**[Table 1]**

| Kinase Inhibiting Activity (IC₅₀µM) | | | |
|---|---|---|---|
| Kinase | Compound4 | Example1 | Example2 |
| ROCKII | 0.01 | 0.0321 | 0. 0290 |
| PKA | 3. 03 | >100 | >100 |
| PKC | 5. 68 | >100 | >100 |
| PKG | 0. 36 | 63. 5 | 28.0 |
| M LC K | 28. 3 | >100 | >100 |
| CaMKII | 0. 18 | 22. 9 | 26. 7 |
| AuroraA | 0. 75 | 84. 5 | 92. 2 |
| MSK1 | 1 <10 | 10<100 | 10<100 |
| MKK4 | 16.9 | >100 | >100 |
| Abl | 7. 77 | >100 | > 100 |
| Src | 3. 06 | >100 | > 100 |

The ROCKII-inhibiting activity of a compound to be tested was defined as 1.0, and the inhibitory activities of the compound against kinases (PKA, PKG, CaMKII, and AuroraA) other than ROCKII were calculated as relative ratios to the ROCKII-inhibiting activity, based on the IC₅₀ values calculated using the above-described evaluation system. Table 2 shows the comparison results.

**[Table 2]**

| Realative Kinase-inhibiting Activity | | | | | |
|---|---|---|---|---|---|
| Compound | Realative Inhibitory Activity | | | | |
| | ROCKII | PKA | PKG | CaMKII | AuroraA |
| Example 1 | 1 | >3000 | 2117 | 763 | 2817 |
| Example2 | 1 | >3000 | 933 | 890 | >3000 |
| Example3 | 1 | >300 | 11 | 135 | 145 |
| Example4 | 1 | >300 | 93 | 300 | >300 |
| Example6 | 1 | >300 | 30 | >300 | >300 |
| Example9 | 1 | >160 | 46 | 8 3 | 201 |
| Example 11 | 1 | >300 | 79 | 364 | 210 |
| Example 12 | 1 | >300 | 92 | >300 | 244 |
| Compund4 | 1 | 300 | 36 | 18 | 75 |

In the above Tables, ROCKII, PKA, PKG, CaMKII, and AuroraA represent the same meanings as described above.
Specific preparation examples will be shown below.

### Preparation Example 1 (Capsule)

| | |
|---|---|
| Compound of Example 1 | 30 mg |
| Microcrystalline cellulose | 30 mg |
| Lactose | 57 mg |
| Magnesium stearate | 3 mg |
| Total | 120 mg |

The above ingredients were mixed in a conventional manner, and a gelatin capsule was filled with the mixture to give a capsule.

### Preparation Example 2 (Tablet)

| | |
|---|---|
| Compound of Example 1 | 30 mg |
| Starch | 44 mg |
| Starch (for paste) | 5.6 mg |
| Magnesium stearate | 0.4 mg |
| Carboxymethylcellulose calcium | 20 mg |
| Total | 100 mg |

The above ingredients were mixed in a conventional manner to give a tablet.

### Preparation Example 3 (Injection)

The compound (100 mg) of Example 1 and sodium chloride (900 mg) were dissolved in about 80 mL of distilled water for injection, and distilled water for injection was added thereto to adjust the total volume to 100 mL. The resulting solution was aseptically filtered and then divided into 10 ampoules. The ampoules were sealed to give aseptic injections.

## Claims

1. A homopiperazine derivative represented by General Formula (1), an acid addition salt thereof, or a solvate of the derivative or the salt: 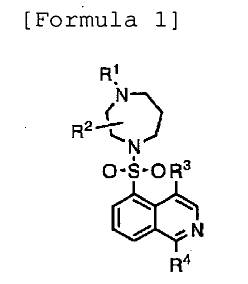 (wherein R¹ represents an amino acid residue; R² represents a hydrogen atom or an alkyl group; R³ represents a hydrogen atom, an alkyl group, an alkenyl group, an alkynyl group, an alkoxy group, a halogen atom, a nitrile group, or a hydroxyl group; and R⁴ represents a hydrogen atom, a halogen atom, or a hydroxyl group).

2. A homopiperazine derivative according to claim 1, an acid addition salt thereof, or a solvate of the derivative or the salt, wherein the amino acid residue is a group selected from the group consisting of glycyl, sarcosyl, alanyl, 1-aminocyclopropane-1-carbonyl, 1-aminocyclobutane-1-carbonyl, β-alanyl, γ-aminobutyroyl, α-aminobutyryl, valyl, norvalyl, leucyl, isoleucyl, norleucyl, tert-leucyl, phenylalanyl, homophenylalanyl, aspartyl, glutamyl, α,β-diaminopropionyl, α,γ-diaminobutyroyl, ornityl, lysyl, arginyl, homoarginyl, seryl, threonyl, tyrosyl, prolyl, triptophyl, and histidyl, the amino acid residue being in L- or D-configuration or a racemic mixture when the amino acid residue has an asymmetric carbon.

3. A homopiperazine derivative according to claim 1, an acid addition salt thereof, or a solvate of the derivative or the salt, wherein the amino acid residue is a glycyl or sarcosyl group.

4. A homopiperazine derivative according to any one of claims 1 to 3, an acid addition salt thereof, or a solvate of the derivative or the salt, wherein R² represents 2-(S)-methyl.

5. A homopiperazine derivative according to claim 1, selected from the group consisting of 2-(S)-1-(4-fluoroisoquinoline-5-sulfonyl)-4-glycyl-2-methylhomopiperazine, 2-(S)-1-(4-fluoroisoquinoline-5-sulfonyl)-2-methyl-4-(N-methylglycyl)homopiperazine, 2-(S)-4-(L-alanyl)-1-(4-fluoroisoquinoline-5-sulfonyl)-2-methylhomopiperazine, 2-(S)-4-(D-alanyl)-1-(4-fluoroisoquinoline-5-sulfonyl)-2-methylhomopiperazine, 2-(S)-1-(4-fluoroisoquinoline-5-sulfonyl)-2-methyl-4-(L-valyl)homopiperazine, 2-(S)-1-(4-fluoroisoquinoline-5-sulfonyl)-2-methyl-4-(D-valyl)homopiperazine, 2-(S)-1-(4-fluoroisoquinoline-5-sulfonyl)-4-(L-leucyl)-2-methylhomopiperazine, 2-(S)-1-(4-fluoroisoquinoline-5-sulfonyl)-4-(D-leucyl)-2-methylhomopiperazine, 2-(S)-4-(β-alanyl)-1-(4-fluoroisoquinoline-5-sulfonyl)-2-methylhomopiperazine, 2-(S)-4-(1-aminocyclopropane-1-carbonyl)-1-(4-fluoroisoquinoline-5 -sulfonyl)-2-methylhomopiperazine, 2-(S)-4-(L-α-aminobutyryl)-1-(4-fluoroisoquinoline-5-sulfonyl)-2-methylhomopiperazine, 2-(S)-4-(D-α-aminobutyryl)-1-(4-fluoroisoquinoline-5-sulfonyl)-2-methylhomopiperazine, 2-(S)-1-(4-fluoroisoquinoline-5-sulfonyl)-2-methyl-4-(L-norleucyl)homopiperazine, 2-(S)-1-(4-fluoroisoquinoline-5-sulfonyl)-2-methyl-4-(D-norleucyl)homopiperazine, 4-(glycyl)-1-(isoquinoline-5-sulfonyl)homopiperazine, 4-β-alanyl)-1-(isoquinoline-5-sulfonyl)homopiperazine, 4-(L-alanyl)-1-(isoquinoline-5-sulfonyl)homopiperazine, 4-(D-alanyl)-1-(isoquinoline-5-sulfonyl)homopiperazine, 1-(isoquinoline-5-sulfonyl)-4-(L-valyl)homopiperazine, 1-(isoquinoline-5-sulfonyl)-4-(D-valyl)homopiperazine, 4-(L-α,β-diaminopropionyl)-1-(isoquinoline-5-sulfonyl)homopiperazine, 4-(D-α,β-diaminopropionyl)-1-(isoquinoline-5-sulfonyl)homopiperazine, 2-(S)-4-(L-α,β-diaminopropionyl)-1-(4-fluoroisoquinoline-5-sulfonyl)-2-methylhomopiperazine, 2-(S)-4-(D-α,β-diaminopropionyl)-1-(4-fluoroisoquinoline-5-sulfonyl)-2-methylhomopiperazine, 4-(glycyl)-1-(4-methylisoquinoline-5-sulfonyl)homopiperazine, 2-(S)-4-(glycyl)-1-(4-methylisoquinoline-5-sulfonyl)-2-methylhomopiperazine, 2-(S)-1-(4-bromoisoquinoline-5-sulfonyl)-4-(glycyl)-2-methylhomopiperazine, and 2-(S)-1-(4-ethenylisoquinoline-5-sulfonyl)-4-(glycyl)-2-methylhomopiperazine; acid addition salts of the above compounds, and solvates of the above compounds or the salts.

6. A drug comprising the homopiperazine derivative according to any one of claims 1 to 5, an acid salt thereof, or a solvate of the derivative or the salt, as an active ingredient.

7. A pharmaceutical composition comprising the homopiperazine derivative according to any one of claims 1 to 5, an acid addition salt thereof, or a solvate of the derivative or the acid addition salt thereof, and a pharmaceutically acceptable carrier.

8. A homopiperazine derivative according to any of claims 1 to 5, for use in medical treatment.

9. A homopiperazine derivative according to claim 8, for use in treating a disease caused by activation of Rho-kinase.

10. A homopiperazine derivative according to claim 8, for use in treating hypertension, pulmonary hypertension, cerebral vasospasm, cardiac angina, cardiac failure, arteriosclerosis, glaucoma, dysuria, asthma, or erectile dysfunction.

11. Use of a homopiperazine derivative according to any one of claims 1 to 5, an acid addition salt thereof, or a solvate of the derivative or the salt, in the manufacture of a drug.

12. Use according to claim 11, wherein the drug is a therapeutic agent for a disease caused by activation of Rho-kinase.

13. Use according to claim 11, wherein the drug is a therapeutic agent for the treatment of hypertension, pulmonary hypertension, cerebral vasospasm, cardiac angina, cardiac failure, arteriosclerosis, glaucoma, dysuria, asthma, or erectile dysfunction.

## Patentansprüche

1. Homopiperazin-Derivat dargestellt durch die allgemeine Formel (1), ein Säureadditionssalz davon oder ein Solvat des Derivats oder des Salzes: 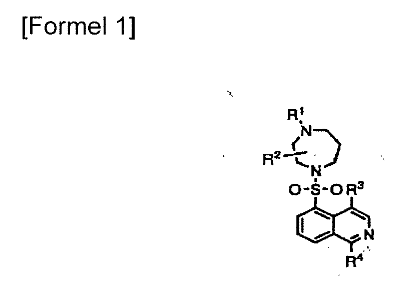 (worin R¹ ein Aminosäurerest ist; R² ein Wasserstoffatom oder eine Alkylgruppe ist; R³ ein Wasserstoffatom, eine Alkylgruppe, eine Alkenylgruppe, eine Alkinylgruppe, eine Alkoxygruppe, ein Halogenatom, eine Nitrilgruppe oder eine Hydroxylgruppe ist; und R⁴ ein Wasserstoffatom, ein Halogenatom oder eine Hydroxylgruppe ist).

2. Homopiperazin-Derivat nach Anspruch 1, ein Säureadditionssalz davon oder ein Solvat des Derivats oder des Salzes, worin der Aminosäurerest eine Gruppe ist, die ausgewählt ist aus der Gruppe bestehend aus Glycyl, Sarcosyl, Alanyl, 1-Aminocyclopropan-1-carbonyl, 1-Aminocyclobutan-1-carbonyl, β-Alanyl, γ-Aminobutyroyl, α-Aminobutyryl, Valyl, Norvalyl, Leucyl, Isoleucyl, Norleucyl, tert.-Leucyl, Phenylalanyl, Homophenylalanyl, Aspartyl, Glutamyl, α,β-Diaminopropionyl, α,γ-Diaminobutyroyl, Ornityl, Lysyl, Arginyl, Homoarginyl, Seryl, Threonyl, Tyrosyl, Prolyl, Triptophyl und Histidyl, wobei der Aminosäurerest in L- oder D-Konfiguration ist oder ein racemische Mischung ist, wenn der Aminosäurerest einen asymmetrischen Kohlenstoff aufweist.

3. Homopiperazin-Derivat nach Anspruch 1, ein Säureadditionssalz davon oder ein Solvat des Derivats oder des Salzes, worin der Aminosäurerest eine Glycyl- oder Sarcosylgruppe ist.

4. Homopiperazin-Derivat nach irgendeinem der Ansprüche 1 bis 3, ein Säureadditionssalz davon oder ein Solvat des Derivats oder des Salzes, worin R² 2-(S)-Methyl ist.

5. Homopiperazin-Derivat nach Anspruch 1, ausgewählt aus der Gruppe bestehend aus 2-(S)-1-(4-Fluorisochinolin-5-sulfonyl)-4-glycyl-2-methylhomopiperazin, 2-(S)-1-(4-Fluorisochinolin-5-sulfonyl)-2-methyl-4-(N-methylglycyl)-homopiperazin, 2-(S)-4-(L-Alanyl)-1-(4-fluorisochinolin-5-sulfonyl)-2-methylhomopiperazin, 2-(S)-4-(D-Alanyl)-1-(4-fluorisochinolin-5-sulfonyl)-2-methylhomopiperazin, 2-(S)-1-(4-Fluorisochinolin-5-sulfonyl)-2-methyl-4-(L-valyl)-homopiperazin, 2-(S)-1-(4-Fluorisochinolin-5-sulfonyl)-2-methyl-4-(D-valyl)-homopiperazin, 2-(S)-1-(4-Fluorisochinolin-5-sulfonyl)-4-(L-leucyl)-2-methylhomopiperazin, 2-(S)-1-(4-Fluorisochinolin-5-sulfonyl)-4-(D-leucyl)-2-methylhomopiperazin, 2-(S)-4-(β-Alanyl)-1-(4-fluorisochinolin-5-sulfonyl)-2-methylhomopiperazin, 2-(S)-4-(1-Aminocyclopropan-1-carbonyl)-1-(4-fluorisochinolin-5-sulfonyl)-2-methylhomopiperazin, 2-(S)-4-(L-α-Aminobutyryl)-1-(4-fluorisochinolin-5-sulfonyl)-2-methylhomopiperazin, 2-(S)-4-(D-α-Aminobutyryl)-1-(4-fluorisochinolin-5-sulfonyl)-2-methylhomopiperazin, 2-(S)-1-(4-Fluorisochinolin-5-sulfonyl)-2-methyl-4-(L-norleucyl)homopiperazin, 2-(S)-1-(4-Fluorisochinolin-5-sulfonyl)-2-methyl-4-(D-norleucyl)homopiperazin, 4-(Glycyl)-1-(isochinolin-5-sulfonyl)homopiperazin, 4-(β-Alanyl)-1-(isochinolin-5-sulfonyl)-homopiperazin, 4-(L-Alanyl)-1-(isochinolin-5-sulfonyl)homopiperazin, 4-(D-Alanyl)-1-(isochinolin-5-sulfonyl)homopiperazin, 1-(Isochinolin-5-sulfonyl)-4-(L-valyl)homopiperazin, 1-(lsochinolin-5-sulfonyl)-4-(D-valyl)homopiperazin, 4-(L-α,ß-Diaminopropionyl)-1-(isochinolin-5-sulfonyl)homopiperazin, 4-(D-α,β-Diaminopropionyl)-1-(isochinolin-5-sulfonyl)homopiperazin, 2-(S)-4-(L-α,β-Diaminopropionyl)-1-(4-fluorisochinolin-5-sulfonyl)-2-methylhomopiperazin, 2-(S)-4-(D- α,β-Diaminopropionyl)-1-(4-fluorisochinolin-5-sulfonyl)-2-methylhomopiperazin, 4-(Glycyl)-1-(4-methylisochinolin-5-sulfonyl)homopiperazin, 2-(S)-4-(Glycyl)-1-(4-methylisochinolin-5-sulfonyl)-2-methylhomopiperazin, 2-(S)-1-(4-Bromisochinolin-5-sulfonyl)-4-(glycyl)-2-methylhomopiperazin und 2-(S)-1-(4-Ethenylisochinolin-5-sulfonyl)-4-(glycyl)-2-methylhomopiperazin; Säureadditionssalze der oben genannten Verbindungen und Solvate der oben genannten Verbindungen oder der Salze.

6. Arzneimittel, umfassend das Homopiperazin-Derivat nach irgendeinem der Ansprüche 1 bis 5, ein Säureadditionssalz davon oder ein Solvat des Derivats oder des Salzes als Wirkbestandteil.

7. Pharmazeutische Zusammensetzung, umfassend das Homopiperazin-Derivat nach irgendeinem der Ansprüche 1 bis 5, ein Säureadditionssalz davon oder ein Solvat des Derivats oder des Säureadditionssalzes davon und einen pharmazeutisch akzeptablen Träger.

8. Homopiperazin-Derivat nach irgendeinem der Ansprüche 1 bis 5 zur Verwendung in der medizinischen Behandlung.

9. Homopiperazin-Derivat nach Anspruch 8 zur Verwendung bei der Behandlung einer Erkrankung, die durch Aktivierung von Rho-Kinase verursacht ist.

10. Homopiperazin-Derivat nach Anspruch 8 zur Verwendung bei der Behandlung von Hypertonie, pulmonaler Hypertonie, zerebralem Vasospasmus, Angina pectoris, Herzversagen, Arteriosklerose, Glaukom, Dysurie, Asthma oder Erektionsstörung.

11. Verwendung eines Homopiperazin-Derivats nach irgendeinem der Ansprüche 1 bis 5, eines Säureadditionssalzes davon oder eines Solvats des Derivats oder des Salzes für die Herstellung eines Arzneimittels.

12. Verwendung nach Anspruch 11, wobei das Arzneimittel ein Therapeutikum gegen eine Erkrankung ist, die durch Aktivierung von Rho-Kinase verursacht ist.

13. Verwendung nach Anspruch 11, wobei das Arzneimittel ein Therapeutikum für die Behandlung von Hypertonie, pulmonaler Hypertonie, zerebralem Vasospasmus, Angina pectoris, Herzversagen, Arteriosklerose, Glaukom, Dysurie, Asthma oder Erektionsstörung ist.

## Revendications

1. Dérivé d'homopipérazine représenté par la formule générale (1), un sel d'addition d'acide de celui-ci, ou un solvate du dérivé ou du sel : 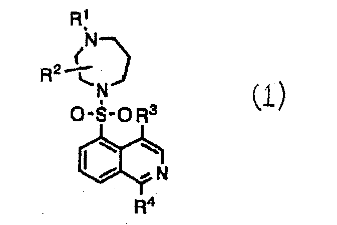 (dans laquelle R¹ représente un résidu d'acide aminé ; R² représente un atome d'hydrogène ou un groupe alkyle ; R³ représente un atome d'hydrogène, un groupe alkyle, un groupe alcényle, un groupe alcynyle, un groupe alcoxy, un atome d'halogène, un groupe nitrile, ou un groupe hydroxyle; et R⁴ représente un atome d'hydrogène, un atome d'halogène, ou un groupe hydroxyle).

2. Dérivé d'homopipérazine selon la revendication 1, un sel d'addition d'acide de celui-ci, ou un solvate du dérivé ou du sel, dans lequel le résidu d'acide aminé est un groupe choisi dans le groupe constitué par le glycyle, le sarcosyle, l'alanyle, le 1-aminocyclopropane-1-carbonyle, le 1-aminocyclobutane-1-carbonyle, le β-alanyle, le γ-aminobutyroyle, l'α-aminobutyryle, le valyle, le norvalyle, le leucyle, l'isoleucyle, le norleucyle, le *tert*-leucyle, le phénylalanyle, l'homophénylalanyle, l'aspartyle, le glutamyle, l'α,β-diaminopropionyle, l'α,γ-diaminobutyroyle, l'ornityle, le lysyle, l'arginyle, l'homoarginyle, le séryle, le thréonyle, le tyrosyle, le propyle, le triptophyle, et l'histidyle, le résidu d'acide aminé étant dans la configuration L ou D ou bien un mélange racémique lorsque le résidu d'acide aminé possède un carbone asymétrique.

3. Dérivé d'homopipérazine selon la revendication 1, un sel d'addition d'acide de celui-ci, ou un solvate du dérivé ou du sel, dans lequel le résidu d'acide aminé est un groupe glycyle ou sarcosyle.

4. Dérivé d'homopipérazine selon l'une quelconque des revendications 1 à 3, un sel d'addition d'acide de celui-ci, ou un solvate du dérivé ou du sel, dans lequel R² représente le 2-(S)-méthyle.

5. Dérivé d'homopipérazine selon la revendication 1, choisi dans le groupe constitué par la 2-(S)-1-(4-fluoroisoquinoléine-5-sulfonyl)-4-glycyl-2-méthylhomopipérazine, 2-(S)-1-(4-fluoroisoquinoléine-5-sulfonyl)-2-méthyl-4-(N-méthylglycyl)homopipérazine, 2-(S)-4-(L-alanyl)-1-(4-fluoroisoquinoléine-5-sulfonyl)-2-méthylhomopipérazine, 2-(S)-4-(D-alanyl)-1-(4-fluoroisoquinoléine-5-sulfonyl)-2-méthylhomopipérazine, 2-(S)-1-(4-fluoroisoquinoléine-5-sulfonyl)-2-méthyl-4-(L-valyl)homopipérazine, 2-(S)-1-(4-fluoroisoquinoléine-S-sulfonyl)-2-méthyl-4-(D-valyl)homopipérazine, 2-(S)-1-(4-fluoroisoquinoléine-5-sulfonyl)-4-(L-leucyl)-2-méthylhomopipérazine, 2-(S)-1-(4-fluoroisoquinoléine-5-sulfonyl)-4-(D-leucyl)-2-méthylhomopipérazine, 2-(S)-4-(β-alanyl)-1-(4-fluoroisoquinoléine-5-sulfonyl)-2-méthylhomopipérazine, 2-(S)-4-(1-aminocyclopropane-1-carbonyl)-1-(4-fluoroisoquinoléine-5-sulfonyl)-2-méthylhomopipérazine, 2-(S)-4-(L-α-aminobutyryl)-1-(4-fluoroisoquinoléine-5-sulfonyl)-2-méthylhomopipérazine, 2-(S)-4-(D-α-aminobutyryl) -1- (4-fluoroisoquinoléine-5-sulfonyl)-2-méthylhomopipérazine, 2-(S)-1-(4-fluoroisoquinoléine-5-sulfonyl)-2-méthyl-4-(L-norleucyl)homopipérazine, 2-(S)-1-(4-fluoroisoquinoléine-5-sulfonyl)-2-méthyl-4-(D-norleucyl)homopipérazine, 4-(glycyl)-1-(isoquinoléine-5-sulfonyl)homopipérazine, 4-(β-alanyl)-1-(isoquinoléine-5-sulfonyl)homopipérazine, 4-(L-alanyl)-1-(isoquinoléine-5-sulfonyl)homopipérazine, 4-(D-alanyl)-1-(isoquinoléine-5-sulfonyl)homopipérazine, 1-(isoquinoléine-5-sulfonyl)-4-(L-valyl)homopipérazine, 1-(isoquinoléine-5-sulfonyl)-4-(D-valyl)homopipérazine, 4-(L-α,β-diaminopropionyl)-1-(isoquinoléine-5-sulfonyl)homopipérazine, 4-(D-α,β-diaminopropionyl)-1-(isoquinoléine-5-sulfonyl)homopipérazine, 2-(S)-4-(L-α,β-diaminopropionyl)-1-(4-fluoroisoquinoléine-5-sulfonyl)-2-méthylhomopipérazine, 2-(S)-4-(D-α,β-diaminopropionyl)-1-(4-fluoroisoquinoléine-5-sulfonyl)-2-méthylhomopipérazine, 4-(glycyl)-1-(4-méthylisoquinoléine-5-sulfonyl)homopipérazine, 2-(S)-4-(glycyl)-1-(4-méthylisoquinoléine-5-sulfonyl)-2-méthylhomopipérazine, 2-(S)-1-(4-bromoisoquinoléine-5-sulfonyl)-4-(glycyl)-2-méthylhomopipérazine, et 2-(S)-1-(4-éthénylisoquinoléine-5-sulfonyl)-4-(glycyl)-2-mêthylhomopipérazine ; les sels d'addition d'acide des composés ci-dessus, et les solvates des composés ci-dessus ou des sels.

6. Médicament comprenant le dérivé d'homopipérazine selon l'une quelconque des revendications 1 à 5, un sel acide de celui-ci, ou un solvate du dérivé ou du sel, comme ingrédient actif.

7. Composition pharmaceutique comprenant le dérivé d'homopipérazine selon l'une quelconque des revendications 1 à 5, un sel d'addition d'acide de celui-ci, ou un solvate du dérivé ou du sel d'addition d'acide de celui-ci, et un support pharmaceutiquement acceptable.

8. Dérivé d'homopipérazine selon l'une quelconque des revendications 1 à 5, pour une utilisation dans un traitement médical.

9. Dérivé d'homopipérazine selon la revendication 8, pour une utilisation dans le traitement d'une maladie provoquée par l'activation de la Rho-kinase.

10. Dérivé d'homopipérazine selon la revendication 8, pour une utilisation dans le traitement d'une hypertension, d'une hypertension pulmonaire, d'un vasospasme cérébral, d'une angine de poitrine, d'une insuffisance cardiaque, d'une artériosclérose, d'un glaucome, d'une dysurie, d'un asthme, ou d'une dysfonction érectile.

11. Utilisation d'un dérivé d'homopipérazine selon l'une quelconque des revendications 1 à 5, d'un sel d'addition d'acide de celui-ci, ou d'un solvate du dérivé ou du sel, dans la fabrication d'un médicament.

12. Utilisation selon la revendication 11, dans laquelle le médicament est un agent thérapeutique pour une maladie provoquée par l'activation de la Rho-kinase.

13. Utilisation selon la revendication 11, dans laquelle le médicament est un agent thérapeutique pour le traitement d'une hypertension, d'une hypertension pulmonaire, d'un vasospasme cérébral, d'une angine de poitrine, d'une insuffisance cardiaque, d'une artériosclérose, d'un glaucome, d'une dysurie, d'un asthme, ou d'une dysfonction érectile.
